# EUROPÄISCHE PATENTSCHRIFT

(11) **EP 1 402 034 B1**
(45) Veröffentlichungstag und Bekanntmachung des Hinweises auf die Patenterteilung: **06.06.2007**
(21) Anmeldenummer: 02740748.5
(22) Anmeldetag: 24.06.2002
(51) Int. Cl.: C12N 15/12, C07K 14/435, A01K 67/033, A61K 31/7088

(54) **IDENTIFIZIERUNG VON SES-1 AUS C. ELEGANS SOWIE DESSEN VERWENDUNG**
IDENTIFICATION OF SES-1 AND THE USES OF THE SAME
IDENTIFICATION DE SES1-1 ET LEURS UTILISATIONS

(30) Priorität: 22.06.2001 DE 10130166
(43) Veröffentlichungstag der Anmeldung: 31.03.2004
(73) Patentinhaber: Sanofi-Aventis Deutschland GmbH, 65929 Frankfurt am Main (DE)
(72) Erfinder: BAUMEISTER, Ralf, 82194 Gröbenzell (DE); LAKOWSKI, Bernard, 81375 München (DE)
(86) Internationale Anmeldenummer: PCT/EP2002/006965
(87) Internationale Veröffentlichungsnummer: WO 2003/000889

(56) Entgegenhaltungen:
- WO-A-00/24880
- WO-A-97/11956
- DATABASE EMBL [Online] 9. Dezember 1995 (1995-12-09) Database accession no. U41542 XP002250916
- WEN CHENHUI ET AL: "spr-2, a suppressor of the egg-laying defect caused by loss of sel-12 presenilin in Caenorhabditis elegans, is a member of the SET protein subfamily." PROCEEDINGS OF THE NATIONAL ACADEMY OF SCIENCES OF THE UNITED STATES, Bd. 97, Nr. 26, 19. Dezember 2000 (2000-12-19), Seiten 14524-14529, XP002247707 December 19, 2000 ISSN: 0027-8424
- REN R F ET AL: "Differential effects of transforming growth factor-betas and glial cell line-derived neurotrophic factor on gene expression of presenilin-1 in human post-mitotic neurons and astrocytes." NEUROSCIENCE, Bd. 93, Nr. 3, 4. August 1999 (1999-08-04), Seiten 1041-1049, XP002250915 ISSN: 0306-4522
- EIMER S ET AL: "Loss of spr-5 bypasses the requirement for the C. elegans presenilin sel-12 by derepressing hop-1." EMBO (EUROPEAN MOLECULAR BIOLOGY ORGANIZATION) JOURNAL, Bd. 21, Nr. 21, 1. November 2002 (2002-11-01), Seiten 5787-5796, XP002247754 ISSN: 0261-4189

### Sequenzprotokoll

<110> EleGene AG
<120> Identifizierung von *ses-1* sowie dessen Verwendungen
<130> 88 839
<160> 7
<210> 1
   <211> 684
   <212> PRT, Einbuchstabencode
   <213> Caenorhabditit elegans
<223> Lange Form der SES-1 Aminosäuresequenz
<400> 1
<210> 2
   <211> 623
   <212> PRT, Einbuchstabencode
   <213> Caenorhabditit elegans
<223> Kurze Form der SES-1 Aminosäuresequenz
<400> 2
<210> 3
   <211> 2055
   <212> cDNA
   <213> Caenorhabditit elegans
<223> Lange Form der cDNA *ses-1* von ATG bis Stop (yk64e9) (Sequenz in Kleinbuchstaben entspricht dem Bereich, der nur in der langen Form vorkommt)
<400> 3
   ATGCCACCTCGCAAGCGCAAACTTGAGGAGTTGAAGAATGAACAAGGAGAGCAGGGGTCATACAAATGCCATCTT
   TGTGGACAGTGCTTCTACCGTGGATGTGGATTAGCAAGTCACCTGCGACGACACGCACCAGTCACATTTGACTGT
   GAGCATTGTGCTTACACGTGTAAGCACAAATATGCGTATGATCGTCATCTGTTGCAATCGCATCCTGAGCTCGTG
   GAATCTGGCCCACTAGTTCGAATGTTTAAGAATGAAGATGAAGATGCTCCAATGCCGATTCTCGAGCGAGAAGCA
   GATGTATCCGATGATGCCGTCAAGTATGAGGCACTCTCTTCTTTCTCGGAACCTGAACCAATCAGCTACAAATGT
   CCATTGTGTATATCCACGTTTGGCAGTCACGCTAGAGCAGTCTACCATATTTTGTCACACCGTGTCAAGTTGTAT
   CAAGCACCGAAAAGCCTGAAATTCTTCTCGAGGAAAACAATGCAAGCTCTTGGAGGATTTCGTTTAGAGTCGCAA
   CTGATGATCAAATGGAGATTACAATACGATAATCGAAGTGTGGACGAGAAGCGAACACGGTTATGGCGGTATATG
   GAAGAAAATTTTGGACATGAACATCTGAAGCAACCAAAGCTGGAAAGTGATAATCCTTCGACAAGTAGCTCTTTT
   GAATCACAAACAAACTTGAACGATTCTTCGATAATCAAAAAGAAACTTGTGATGAAGTGTGGTACTGTTTTCGGT
   CAAAGACTCATTCACGACAACACCCAATATTATTTGTGCCGCAATTGCCCATACGTTTCGTGGAATGTTTCTTCA
   CTGTGGCGACATTTCCGTCATCATATTCAAAAATCGAAACAATCTTGGACGTGTATCGCATGCTCATACAGCTCA
   TCGAGCCGGGTGAAAATTGATCTACATGTGAAAATGCATAAGGAAATGCCGGAAATTGATTTGGAGTTTGCAACT
   TGGCTTCGCTATGAACGAAGAATCAACAAGAACGACTTGAATAAACCGACTAACAAAAAAAAGAAACCCGATGGA
   GGAAACGGTTCAAATCACAGTGATATGAGAAGTCTTCACGCATTTCTTAGTCTTAAAAATTCTAAGAACAATGTT
   GTCAAACACGATATCGATGCTCCAACTTTGCACCCATTATCCCCAGCGCCCAAGCTGGTCGCGATGACTCAATTT
   GACTTCGGAGAAATTGTCACTTACAAAAGCGTCAATCCGCTGCACCAAATCAACAAGAACAATAGCAATCCGACG
   GTCCTGCCGAACAAGCGAAATAGCATTAAAACCAgtaaaagtgatactcaaatcgcgctcagcgttaagcagtct
   agtagtatgaagatggtgaaagtttctcctggaaaagtttatcagcttcctaagacgtccaaattttaccgacct
   gaatcacctgactcattagcatcaaataattctgctcacggagacgagattgagtcaacgtcttcagATCAATTC
   CAACAATCCGTGAAAGTTCCAAAGTACGAAGACTTTTTGAACATGAAGCCAGTTATGCCATATTTCCAAAAACAA
   CGACATCCACTGGAAGCGATTGCTATGTACGAAAAGGCAAAAAGGGAATACGAAAAGAATCACTGTTTCCCGAAT
   CTTCCAATTTTCGAATTCAACATTGAGTACAAGAACTTGCATCCACTCGCCAAGGCACAGTATGGGAAAAACAAT
   ATGAAAGAGTATTTTTTGAATGAAATGGAAGTAGAGAAATCTCGCGAATGCACTGACTGCCCATTCAAACACAAC
   GATCTGCAGCAGTTCCGTCTCCACCGTGACAAGCATTTCTACGGTGGATCACACACCTGTCCGGAGTGCAACTAT
   TCATCGAACAACCATAATCAAGTGGTCGAGCACACGTTTGTGGATCACTATTTGAGCGATGTGAGATTGGTTGAA
   GGGCTTCCGTCTTCGGATTCGGAGGACGATAATATTCCGGTACCTCCTGATACTCCGCAGCGGAAGAAAAAAGCG
   CCGAAGCGTGGCAAACGTCGTGGCTGGTAA
<210> 4
   <211> 2055 + 442
   <212> cDNA
   <213> Caenorhabditit elegans
<223> Lange Form der cDNA *ses-1* von ATG bis Stop (yk64e9) + nichtkodierende Region 3'Ende (Sequenz in Kleinbuchstaben entspricht dem Bereich, der nur in der langen Form vorkommt)
<400> 4
<210> 5
   <211> 1872
   <212> cDNA
   <213> Caenorhabditit elegans
<223> Kurze Form der cDNA *ses-1* von ATG bis Stop (yk247e5)
<400> 5
   ATGCCACCTCGCAAGCGCAAACTTGAGGAGTTGAAGAATGAACAAGGAGAGCAGGGGTCATACAAATGCCATCTT
   TGTGGACAGTGCTTCTACCGTGGATGTGGATTAGCAAGTCACCTGCGACGACACGCACCAGTCACATTTGACTGT
   GAGCATTGTGCTTACACGTGTAAGCACAAATATGCGTATGATCGTCATCTGTTGCAATCGCATCCTGAGCTCGTG
   GAATCTGGCCCACTAGTTCGAATGTTTAAGAATGAAGATGAAGATGCTCCAATGCCGATTCTCGAGCGAGAAGCA
   GATGTATCCGATGATGCCGTCAAGTATGAGGCACTCTCTTCTTTCTCGGAACCTGAACCAATCAGCTACAAATGT
   CCATTGTGTATATCCACGTTTGGCAGTCACGCTAGAGCAGTCTACCATATTTTGTCACACCGTGTCAAGTTGTAT
   CAAGCACCGAAAAGCCTGAAATTCTTCTCGAGGAAAACAATGCAAGCTCTTGGAGGATTTCGTTTAGAGTCGCAA
   CTGATGATCAAATGGAGATTACAATACGATAATCGAAGTGTGGACGAGAAGCGAACACGGTTATGGCGGTATATG
   GAAGAAAATTTTGGACATGAACATCTGAAGCAACCAAAGCTGGAAAGTGATAATCCTTCGACAAGTAGCTCTTTT
   GAATCACAAACAAACTTGAACGATTCTTCGATAATCAAAAAGAAACTTGTGATGAAGTGTGGTACTGTTTTCGGT
   CAAAGACTCATTCACGACAACACCCAATATTATTTGTGCCGCAATTGCCCATACGTTTCGTGGAATGTTTCTTCA
   CTGTGGCGACATTTCCGTCATCATATTCAAAAATCGAAACAATCTTGGACGTGTATCGCATGCTCATACAGCTCA
   TCGAGCCGGGTGAAAATTGATCTACATGTGAAAATGCATAAGGAAATGCCGGAAATTGATTTGGAGTTTGCAACT
   TGGCTTCGCTATGAACGAAGAATCAACAAGAACGACTTGAATAAACCGACTAACAAAAAAAAGAAACCCGATGGA
   GGAAACGGTTCAAATCACAGTGATATGAGAAGTCTTCACGCATTTCTTAGTCTTAAAAATTCTAAGAACAATGTT
   GTCAAACACGATATCGATGCTCCAACTTTGCACCCATTATCCCCAGCGCCCAAGCTGGTCGCGATGACTCAATTT
   GACTTCGGAGAAATTGTCACTTACAAAAGCGTCAATCCGCTGCACCAAATCAACAAGAACAATAGCAATCCGACG
   GTCCTGCCGAACAAGCGAAATAGCATTAAAACCAATCAATTCCAACAATCCGTGAAAGTTCCAAAGTACGAAGAC
   TTTTTGAACATGAAGCCAGTTATGCCATATTTCCAAAAACAACGACATCCACTGGAAGCGATTGCTATGTACGAA
   AAGGCAAAAAGGGAATACGAAAAGAATCACTGTTTCCCGAATCTTCCAATTTTCGAATTCAACATTGAGTACAAG
   AACTTGCATCCACTCGCCAAGGCACAGTATGGGAAAAACAATATGAAAGAGTATTTTTTGAATGAAATGGAAGTA
   GAGAAATCTCGCGAATGCACTGACTGCCCATTCAAACACAACGATCTGCAGCAGTTCCGTCTCCACCGTGACAAG
   CATTTCTACGGTGGATCACACACCTGTCCGGAGTGCAACTATTCATCGAACAACCATAATCAAGTGGTCGAGCAC
   ACGTTTGTGGATCACTATTTGAGCGATGTGAGATTGGTTGAAGGGCTTCCGTCTTCGGATTCGGAGGACGATAAT
   ATTCCGGTACCTCCTGATACTCCGCAGCGGAAGAAAAAAGCGCCGAAGCGTGGCAAACGTCGTGGCTGGTAA
<210> 6
   <211> 1872 + 442
   <212> cDNA
   <213> Caenorhabditit elegans
<223> Kurze Form der cDNA *ses-1* von ATG bis Stop (yk247e5) + nichtkodierende Region 3'Ende
<400> 6
<210> 7
   <211> 9260
   <212> DNA
   <213> Caenorhabditit elegans
<223> Sequenz von pBy1015, dieser Abschnitt komplementiert den Defekt der *ses-1* Nullmutante
<400> 7

### SEQUENZPROTOKOLL

<110> EleGene AG
<120> Identifizierung von ses-1 sowie dessen Verwendungen
<130> 1A-86174
<140> 10130166.9
   <141> 2001-06-22
<160> 7
<170> PatentIn Ver. 2.1
<210> 1
   <211> 684
   <212> PRT
   <213> Caenorhabditis elegans
<220>
   <223> Lange Form der SES-1 Aminosäuresequenz
<400> 1
   Met Pro Pro Arg Lys Arg Lys Leu Glu Glu Leu Lys Asn Glu Gln Gly
    1               5                  10                  15
   Glu Gln Gly Ser Tyr Lys Cys His Leu Cys Gly Gln Cys Phe Tyr Arg
               20                  25                  30
   Gly Cys Gly Leu Ala Ser His Leu Arg Arg His Ala Pro Val Thr Phe
           35                  40                  45
   Asp Cys Glu His Cys Ala Tyr Thr Cys Lys His Lys Tyr Ala Tyr Asp
       50                  55                  60
   Arg His Leu Leu Gln Ser His Pro Glu Leu Val Glu Ser Gly Pro Leu
   65                  70                  75                  80
   Val Arg Met Phe Lys Asn Glu Asp Glu Asp Ala Pro Met Pro Ile Leu
                   85                  90                  95
   Glu Arg Glu Ala Asp Val Ser Asp Asp Ala Val Lys Tyr Glu Ala Leu
               100                 105                 110
   Ser Ser Phe Ser Glu Pro Glu Pro Ile Ser Tyr Lys Cys Pro Leu Cys
           115                 120                 125
   Ile Ser Thr Phe Gly Ser His Ala Arg Ala Val Tyr His Ile Leu Ser
   130 135 140
   His Arg Val Lys Leu Tyr Gln Ala Pro Lys Ser Leu Lys Phe Phe Ser
   145 150 155 160
   Arg Lys Thr Met Gln Ala Leu Gly Gly Phe Arg Leu Glu Ser Gln Leu
   165 170 175
   Met Ile Lys Trp Arg Leu Gln Tyr Asp Asn Arg Ser Val Asp Glu Lys
   180 185 190
   Arg Thr Arg Leu Trp Arg Tyr Met Glu Glu Asn Phe Gly His Glu His
   195 200 205
   Leu Lys Gln Pro Lys Leu Glu Ser Asp Asn Pro Ser Thr Ser Ser Ser
   210 215 220
   Phe Glu Ser Gln Thr Asn Leu Asn Asp Ser Ser Ile Ile Lys Lys Lys
   225 230 235 240
   Leu Val Met Lys Cys Gly Thr Val Phe Gly Gln Arg Leu Ile His Asp
   245 250 255
   Asn Thr Gln Tyr Tyr Leu Cys Arg Asn Cys Pro Tyr Val Ser Trp Asn
   260 265 270
   Val Ser Ser Leu Trp Arg His Phe Arg His His Ile Gln Lys Ser Lys
   275 280 285
   Gln Ser Trp Thr Cys Ile Ala Cys Ser Tyr Ser Ser Ser Ser Arg Val
   290 295 300
   Lys Ile Asp Leu His Val Lys Met His Lys Glu Met Pro Glu Ile Asp
   305 310 315 320
   Leu Glu Phe Ala Thr Trp Leu Arg Tyr Glu Arg Arg Ile Asn Lys Asn
   325 330 335
   Asp Leu Asn Lys Pro Thr Asn Lys Lys Lys Lys Pro Asp Gly Gly Asn
   340 345 350
   Gly Ser Asn His Ser Asp Met Arg Ser Leu His Ala Phe Leu Ser Leu
   355 360 365
   Lys Asn Ser Lys Asn Asn Val Val Lys His Asp Ile Asp Ala Pro Thr
   370 375 380
   Leu His Pro Leu Ser Pro Ala Pro Lys Leu Val Ala Met Thr Gln Phe
   385 390 395 400
   Asp Phe Gly Glu Ile Val Thr Tyr Lys Ser Val Asn Pro Leu His Gln
   405 410 415
   Ile Asn Lys Asn Asn Ser Asn Pro Thr Val Leu Pro Asn Lys Arg Asn
   420 425 430
   Ser Ile Lys Thr Ser Lys Ser Asp Thr Gln Ile Ala Leu Ser Val Lys
   435 440 445
   Gln Ser Ser Ser Met Lys Met Val Lys Val Ser Pro Gly Lys Val Tyr
   450 455 460
   Gln Leu Pro Lys Thr Ser Lys Phe Tyr Arg Pro Glu Ser Pro Asp Ser
   465 470 475 480
   Leu Ala Ser Asn Asn Ser Ala His Gly Asp Glu Ile Glu Ser Thr Ser
   485 490 495
   Ser Asp Gln Phe Gln Gln Ser Val Lys Val Pro Lys Tyr Glu Asp Phe
   500 505 510
   Leu Asn Met Lys Pro Val Met Pro Tyr Phe Gln Lys Gln Arg His Pro
   515 520 525
   Leu Glu Ala Ile Ala Met Tyr Glu Lys Ala Lys Arg Glu Tyr Glu Lys
   530 535 540
   Asn His Cys Phe Pro Asn Leu Pro Ile Phe Glu Phe Asn Ile Glu Tyr
   545 550 555 560
   Lys Asn Leu His Pro Leu Ala Lys Ala Gln Tyr Gly Lys Asn Asn Met
   565 570 575
   Lys Glu Tyr Phe Leu Asn Glu Met Glu Val Glu Lys Ser Arg Glu Cys
   580 585 590
   Thr Asp Cys Pro Phe Lys His Asn Asp Leu Gln Gln Phe Arg Leu His
   595 600 605
   Arg Asp Lys His Phe Tyr Gly Gly Ser His Thr Cys Pro Glu Cys Asn
   610 615 620
   Tyr Ser Ser Asn Asn His Asn Gln Val Val Glu His Thr Phe Val Asp
   625 630 635 640
   His Tyr Leu Ser Asp Val Arg Leu Val Glu Gly Leu Pro Ser Ser Asp
   645 650 655
   Ser Glu Asp Asp Asn Ile Pro Val Pro Pro Asp Thr Pro Gln Arg Lys
   660 665 670
   Lys Lys Ala Pro Lys Arg Gly Lys Arg Arg Gly Trp
   675 680
<210> 2
   <211> 623
   <212> PRT
   <213> Caenorhabditis elegans
<220>
   <223> Kurze Form der SES-1 Aminosäuresequenz
<400> 2
   Met Pro Pro Arg Lys Arg Lys Leu Glu Glu Leu Lys Asn Glu Gln Gly
    1               5                  10                  15
   Glu Gln Gly Ser Tyr Lys Cys His Leu Cys Gly Gln Cys Phe Tyr Arg
               20                  25                  30
   Gly Cys Gly Leu Ala Ser His Leu Arg Arg His Ala Pro Val Thr Phe
           35                  40                  45
   Asp Cys Glu His Cys Ala Tyr Thr Cys Lys His Lys Tyr Ala Tyr Asp
       50                  55                  60
   Arg His Leu Leu Gln Ser His Pro Glu Leu Val Glu Ser Gly Pro Leu
   65                  70                  75                  80
   Val Arg Met Phe Lys Asn Glu Asp Glu Asp Ala Pro Met Pro Ile Leu
                   85                  90                  95
   Glu Arg Glu Ala Asp Val Ser Asp Asp Ala Val Lys Tyr Glu Ala Leu
               100                 105                 110
   Ser Ser Phe Ser Glu Pro Glu Pro Ile Ser Tyr Lys Cys Pro Leu Cys
           115                 120                 125
   Ile Ser Thr Phe Gly Ser His Ala Arg Ala Val Tyr His Ile Leu Ser
       130                 135                 140
   His Arg Val Lys Leu Tyr Gln Ala Pro Lys Ser Leu Lys Phe Phe Ser
   145                 150                 155                 160
   Arg Lys Thr Met Gln Ala Leu Gly Gly Phe Arg Leu Glu Ser Gln Leu
   165 170 175
   Met Ile Lys Trp Arg Leu Gln Tyr Asp Asn Arg Ser Val Asp Glu Lys
   180 185 190
   Arg Thr Arg Leu Trp Arg Tyr Met Glu Glu Asn Phe Gly His Glu His
   195 200 205
   Leu Lys Gln Pro Lys Leu Glu Ser Asp Asn Pro Ser Thr Ser Ser Ser
   210 215 220
   Phe Glu Ser Gln Thr Asn Leu Asn Asp Ser Ser Ile Ile Lys Lys Lys
   225 230 235 240
   Leu Val Met Lys Cys Gly Thr Val Phe Gly Gln Arg Leu Ile His Asp
   245 250 255
   Asn Thr Gln Tyr Tyr Leu Cys Arg Asn Cys Pro Tyr Val Ser Trp Asn
   260 265 270
   Val Ser Ser Leu Trp Arg His Phe Arg His His Ile Gln Lys Ser Lys
   275 280 285
   Gln Ser Trp Thr Cys Ile Ala Cys Ser Tyr Ser Ser Ser Ser Arg Val
   290 295 300
   Lys Ile Asp Leu His Val Lys Met His Lys Glu Met Pro Glu Ile Asp
   305 310 315 320
   Leu Glu Phe Ala Thr Trp Leu Arg Tyr Glu Arg Arg Ile Asn Lys Asn
   325 330 335
   Asp Leu Asn Lys Pro Thr Asn Lys Lys Lys Lys Pro Asp Gly Gly Asn
   340 345 350
   Gly Ser Asn His Ser Asp Met Arg Ser Leu His Ala Phe Leu Ser Leu
   355 360 365
   Lys Asn Ser Lys Asn Asn Val Val Lys His Asp Ile Asp Ala Pro Thr
   370 375 380
   Leu His Pro Leu Ser Pro Ala Pro Lys Leu Val Ala Met Thr Gln Phe
   385 390 395 400
   Asp Phe Gly Glu Ile Val Thr Tyr Lys Ser Val Asn Pro Leu His Gln
   405 410 415
   Ile Asn Lys Asn Asn Ser Asn Pro Thr Val Leu Pro Asn Lys Arg Asn
               420                 425                 430
   Ser Ile Lys Thr Asn Gln Phe Gln Gln Ser Val Lys Val Pro Lys Tyr
           435                 440                 445
   Glu Asp Phe Leu Asn Met Lys Pro Val Met Pro Tyr Phe Gln Lys Gln
       450                 455                 460
   Arg His Pro Leu Glu Ala Ile Ala Met Tyr Glu Lys Ala Lys Arg Glu
   465                 470                 475                 480
   Tyr Glu Lys Asn His Cys Phe Pro Asn Leu Pro Ile Phe Glu Phe Asn
                   485                 490                 495
   Ile Glu Tyr Lys Asn Leu His Pro Leu Ala Lys Ala Gln Tyr Gly Lys
               500                 505                 510
   Asn Asn Met Lys Glu Tyr Phe Leu Asn Glu Met Glu Val Glu Lys Ser
           515                 520                 525
   Arg Glu Cys Thr Asp Cys Pro Phe Lys His Asn Asp Leu Gln Gln Phe
       530                 535                 540
   Arg Leu His Arg Asp Lys His Phe Tyr Gly Gly Ser His Thr Cys Pro
   545                 550                 555                 560
   Glu Cys Asn Tyr Ser Ser Asn Asn His Asn Gln Val Val Glu His Thr
                   565                 570                 575
   Phe Val Asp His Tyr Leu Ser Asp Val Arg Leu Val Glu Gly Leu Pro
               580                 585                 590
   Ser Ser Asp Ser Glu Asp Asp Asn Ile Pro Val Pro Pro Asp Thr Pro
           595                 600                 605
   Gln Arg Lys Lys Lys Ala Pro Lys Arg Gly Lys Arg Arg Gly Trp
       610                 615                 620
<210> 3
   <211> 2055
   <212> DNA
   <213> Caenorhabditis elegans
<220>
   <223> Lange Form der cDNA ses-1 von ATG bis Stop (yk64e9)
<220>
   <221> misc_feature
   <222> (1310)..(1492)
   <223> Bereich, der nur in der langen Form vorkommt
<400> 3
   atgccacctc gcaagcgcaa acttgaggag ttgaagaatg aacaaggaga gcaggggtca 60
   tacaaatgcc atctttgtgg acagtgcttc taccgtggat gtggattagc aagtcacctg 120
   cgacgacacg caccagtcac atttgactgt gagcattgtg cttacacgtg taagcacaaa 180
   tatgcgtatg atcgtcatct gttgcaatcg catcctgagc tcgtggaatc tggcccacta 240
   gttcgaatgt ttaagaatga agatgaagat gctccaatgc cgattctcga gcgagaagca 300
   gatgtatccg atgatgccgt caagtatgag gcactctctt ctttctcgga acctgaacca 360
   atcagctaca aatgtccatt gtgtatatcc acgtttggca gtcacgctag agcagtctac 420
   catattttgt cacaccgtgt caagttgtat caagcaccga aaagcctgaa attcttctcg 480
   aggaaaacaa tgcaagctct tggaggattt cgtttagagt cgcaactgat gatcaaatgg 540
   agattacaat acgataatcg aagtgtggac gagaagcgaa cacggttatg gcggtatatg 600
   gaagaaaatt ttggacatga acatctgaag caaccaaagc tggaaagtga taatccttcg 660
   acaagtagct cttttgaatc acaaacaaac ttgaacgatt cttcgataat caaaaagaaa 720
   cttgtgatga agtgtggtac tgttttcggt caaagactca ttcacgacaa cacccaatat 780
   tatttgtgcc gcaattgccc atacgtttcg tggaatgttt cttcactgtg gcgacatttc 840
   cgtcatcata ttcaaaaatc gaaacaatct tggacgtgta tcgcatgctc atacagctca 900
   tcgagccggg tgaaaattga tctacatgtg aaaatgcata aggaaatgcc ggaaattgat 960
   ttggagtttg caacttggct tcgctatgaa cgaagaatca acaagaacga cttgaataaa 1020
   ccgactaaca aaaaaaagaa acccgatgga ggaaacggtt caaatcacag tgatatgaga 1080
   agtcttcacg catttcttag tcttaaaaat tctaagaaca atgttgtcaa acacgatatc 1140
   gatgctccaa ctttgcaccc attatcccca gcgcccaagc tggtcgcgat gactcaattt 1200
   gacttcggag aaattgtcac ttacaaaagc gtcaatccgc tgcaccaaat caacaagaac 1260
   aatagcaatc cgacggtcct gccgaacaag cgaaatagca ttaaaaccag taaaagtgat 1320
   actcaaatcg cgctcagcgt taagcagtct agtagtatga agatggtgaa agtttctcct 1380
   ggaaaagttt atcagcttcc taagacgtcc aaattttacc gacctgaatc acctgactca 1440
   ttagcatcaa ataattctgc tcacggagac gagattgagt caacgtcttc agatcaattc 1500
   caacaatccg tgaaagttcc aaagtacgaa gactttttga acatgaagcc agttatgcca 1560
   tatttccaaa aacaacgaca tccactggaa gcgattgcta tgtacgaaaa ggcaaaaagg 1620
   gaatacgaaa agaatcactg tttcccgaat cttccaattt tcgaattcaa cattgagtac 1680
   aagaacttgc atccactcgc caaggcacag tatgggaaaa acaatatgaa agagtatttt 1740
   ttgaatgaaa tggaagtaga gaaatctcgc gaatgcactg actgcccatt caaacacaac 1800
   gatctgcagc agttccgtct ccaccgtgac aagcatttct acggtggatc acacacctgt 1860
   ccggagtgca actattcatc gaacaaccat aatcaagtgg tcgagcacac gtttgtggat 1920
   cactatttga gcgatgtgag attggttgaa gggcttccgt cttcggattc ggaggacgat 1980
   aatattccgg tacctcctga tactccgcag cggaagaaaa aagcgccgaa gcgtggcaaa 2040
   cgtcgtggct ggtaa                                                  2055
<210> 4
   <211> 2497
   <212> DNA
   <213> Caenorhabditis elegans
<220>
   <223> Lange Form der cDNA ses-1 von ATG bis Stop (yk64e9) + nichtkodierende Region 3'-Ende
<220>
   <221> 3'UTR
   <222> (2056)..(2497)
<220>
   <221> misc_feature
   <222> (1310)..(1492)
   <223> Bereich, der nur in der langen Form vorkommt
<400> 4
   atgccacctc gcaagcgcaa acttgaggag ttgaagaatg aacaaggaga gcaggggtca 60
   tacaaatgcc atctttgtgg acagtgcttc taccgtggat gtggattagc aagtcacctg 120
   cgacgacacg caccagtcac atttgactgt gagcattgtg cttacacgtg taagcacaaa 180
   tatgcgtatg atcgtcatct gttgcaatcg catcctgagc tcgtggaatc tggcccacta 240
   gttcgaatgt ttaagaatga agatgaagat gctccaatgc cgattctcga gcgagaagca 300
   gatgtatccg atgatgccgt caagtatgag gcactctctt ctttctcgga acctgaacca 360
   atcagctaca aatgtccatt gtgtatatcc acgtttggca gtcacgctag agcagtctac 420
   catattttgt cacaccgtgt caagttgtat caagcaccga aaagcctgaa attcttctcg 480
   aggaaaacaa tgcaagctct tggaggattt cgtttagagt cgcaactgat gatcaaatgg 540
   agattacaat acgataatcg aagtgtggac gagaagcgaa cacggttatg gcggtatatg 600
   gaagaaaatt ttggacatga acatctgaag caaccaaagc tggaaagtga taatccttcg 660
   acaagtagct cttttgaatc acaaacaaac ttgaacgatt cttcgataat caaaaagaaa 720
   cttgtgatga agtgtggtac tgttttcggt caaagactca ttcacgacaa cacccaatat 780
   tatttgtgcc gcaattgccc atacgtttcg tggaatgttt cttcactgtg gcgacatttc 840
   cgtcatcata ttcaaaaatc gaaacaatct tggacgtgta tcgcatgctc atacagctca 900
   tcgagccggg tgaaaattga tctacatgtg aaaatgcata aggaaatgcc ggaaattgat 960
   ttggagtttg caacttggct tcgctatgaa cgaagaatca acaagaacga cttgaataaa 1020
   ccgactaaca aaaaaaagaa acccgatgga ggaaacggtt caaatcacag tgatatgaga 1080
   agtcttcacg catttcttag tcttaaaaat tctaagaaca atgttgtcaa acacgatatc 1140
   gatgctccaa ctttgcaccc attatcccca gcgcccaagc tggtcgcgat gactcaattt 1200
   gacttcggag aaattgtcac ttacaaaagc gtcaatccgc tgcaccaaat caacaagaac 1260
   aatagcaatc cgacggtcct gccgaacaag cgaaatagca ttaaaaccag taaaagtgat 1320
   actcaaatcg cgctcagcgt taagcagtct agtagtatga agatggtgaa agtttctcct 1380
   ggaaaagttt atcagcttcc taagacgtcc aaattttacc gacctgaatc acctgactca 1440
   ttagcatcaa ataattctgc tcacggagac gagattgagt caacgtcttc agatcaattc 1500
   caacaatccg tgaaagttcc aaagtacgaa gactttttga acatgaagcc agttatgcca 1560
   tatttccaaa aacaacgaca tccactggaa gcgattgcta tgtacgaaaa ggcaaaaagg 1620
   gaatacgaaa agaatcactg tttcccgaat cttccaattt tcgaattcaa cattgagtac 1680
   aagaacttgc atccactcgc caaggcacag tatgggaaaa acaatatgaa agagtatttt 1740
   ttgaatgaaa tggaagtaga gaaatctcgc gaatgcactg actgcccatt caaacacaac 1800
   gatctgcagc agttccgtct ccaccgtgac aagcatttct acggtggatc acacacctgt 1860
   ccggagtgca actattcatc gaacaaccat aatcaagtgg tcgagcacac gtttgtggat 1920
   cactatttga gcgatgtgag attggttgaa gggcttccgt cttcggattc ggaggacgat 1980
   aatattccgg tacctcctga tactccgcag cggaagaaaa aagcgccgaa gcgtggcaaa 2040
   cgtcgtggct ggtaaagatt atattttatt aaattttatc aagttttttt aaaggtaaat 2100
   gtttaaattt attttaaatt taaatttatt tttgtttctt tttttttcaa ttaattgttt 2160
   ttaagttttt caatgttagg tttgtcgttt aatttttttc aaatttctaa taatattccc 2220
   ttcccttccc atccccgctc aaatagctgc ctcatagttt agtaaatttc taaataatat 2280
   tatttcaata ccatcacata ttgtcagcgc tttctgtcat ttttttaatt ttcattcatc 2340
   ctggtacctc acttttggca cgtgtccaac aaccaccctg acatcaaccc acccggactt 2400
   tctcgactag gtttttttga aattcttccc ccattttatt ttctttgacc attttatgtt 2460
   tgtgttttga atgaaatgtg ttttcgaatt tttaaaa                          2497
<210> 5
   <211> 1872
   <212> DNA
   <213> Caenorhabditis elegans
<220>
   <223> Kurze Form der cDNA ses-1 von ATG bis Stop (yk247e5)
<400> 5
   atgccacctc gcaagcgcaa acttgaggag ttgaagaatg aacaaggaga gcaggggtca 60
   tacaaatgcc atctttgtgg acagtgcttc taccgtggat gtggattagc aagtcacctg 120
   cgacgacacg caccagtcac atttgactgt gagcattgtg cttacacgtg taagcacaaa 180
   tatgcgtatg atcgtcatct gttgcaatcg catcctgagc tcgtggaatc tggcccacta 240
   gttcgaatgt ttaagaatga agatgaagat gctccaatgc cgattctcga gcgagaagca 300
   gatgtatccg atgatgccgt caagtatgag gcactctctt ctttctcgga acctgaacca 360
   atcagctaca aatgtccatt gtgtatatcc acgtttggca gtcacgctag agcagtctac 420
   catattttgt cacaccgtgt caagttgtat caagcaccga aaagcctgaa attcttctcg 480
   aggaaaacaa tgcaagctct tggaggattt cgtttagagt cgcaactgat gatcaaatgg 540
   agattacaat acgataatcg aagtgtggac gagaagcgaa cacggttatg gcggtatatg 600
   gaagaaaatt ttggacatga acatctgaag caaccaaagc tggaaagtga taatccttcg 660
   acaagtagct cttttgaatc acaaacaaac ttgaacgatt cttcgataat caaaaagaaa 720
   cttgtgatga agtgtggtac tgttttcggt caaagactca ttcacgacaa cacccaatat 780
   tatttgtgcc gcaattgccc atacgtttcg tggaatgttt cttcactgtg gcgacatttc 840
   cgtcatcata ttcaaaaatc gaaacaatct tggacgtgta tcgcatgctc atacagctca 900
   tcgagccggg tgaaaattga tctacatgtg aaaatgcata aggaaatgcc ggaaattgat 960
   ttggagtttg caacttggct tcgctatgaa cgaagaatca acaagaacga cttgaataaa 1020
   ccgactaaca aaaaaaagaa acccgatgga ggaaacggtt caaatcacag tgatatgaga 1080
   agtcttcacg catttcttag tcttaaaaat tctaagaaca atgttgtcaa acacgatatc 1140
   gatgctccaa ctttgcaccc attatcccca gcgcccaagc tggtcgcgat gactcaattt 1200
   gacttcggag aaattgtcac ttacaaaagc gtcaatccgc tgcaccaaat caacaagaac 1260
   aatagcaatc cgacggtcct gccgaacaag cgaaatagca ttaaaaccaa tcaattccaa 1320
   caatccgtga aagttccaaa gtacgaagac tttttgaaca tgaagccagt tatgccatat 1380
   ttccaaaaac aacgacatcc actggaagcg attgctatgt acgaaaaggc aaaaagggaa 1440
   tacgaaaaga atcactgttt cccgaatctt ccaattttcg aattcaacat tgagtacaag 1500
   aacttgcatc cactcgccaa ggcacagtat gggaaaaaca atatgaaaga gtattttttg 1560
   aatgaaatgg aagtagagaa atctcgcgaa tgcactgact gcccattcaa acacaacgat 1620
   ctgcagcagt tccgtctcca ccgtgacaag catttctacg gtggatcaca cacctgtccg 1680
   gagtgcaact attcatcgaa caaccataat caagtggtcg agcacacgtt tgtggatcac 1740
   tatttgagcg atgtgagatt ggttgaaggg cttccgtctt cggattcgga ggacgataat 1800
   attccggtac ctcctgatac tccgcagcgg aagaaaaaag cgccgaagcg tggcaaacgt 1860
   cgtggctggt aa 1872
<210> 6
   <211> 2314
   <212> DNA
   <213> Caenorhabditis elegans
<220>
   <223> Kurze Form der cDNA ses-1 von ATG bis Stop (yk247e5) + nichtkodierende Region 3'-Ende
<220>
   <221> 3'UTR
   <222> (1873)..(2314)
<400> 6
   atgccacctc gcaagcgcaa acttgaggag ttgaagaatg aacaaggaga gcaggggtca 60
   tacaaatgcc atctttgtgg acagtgcttc taccgtggat gtggattagc aagtcacctg 120
   cgacgacacg caccagtcac atttgactgt gagcattgtg cttacacgtg taagcacaaa 180
   tatgcgtatg atcgtcatct gttgcaatcg catcctgagc tcgtggaatc tggcccacta 240
   gttcgaatgt ttaagaatga agatgaagat gctccaatgc cgattctcga gcgagaagca 300
   gatgtatccg atgatgccgt caagtatgag gcactctctt ctttctcgga acctgaacca 360
   atcagctaca aatgtccatt gtgtatatcc acgtttggca gtcacgctag agcagtctac 420
   catattttgt cacaccgtgt caagttgtat caagcaccga aaagcctgaa attcttctcg 480
   aggaaaacaa tgcaagctct tggaggattt cgtttagagt cgcaactgat gatcaaatgg 540
   agattacaat acgataatcg aagtgtggac gagaagcgaa cacggttatg gcggtatatg 600
   gaagaaaatt ttggacatga acatctgaag caaccaaagc tggaaagtga taatccttcg 660
   acaagtagct cttttgaatc acaaacaaac ttgaacgatt cttcgataat caaaaagaaa 720
   cttgtgatga agtgtggtac tgttttcggt caaagactca ttcacgacaa cacccaatat 780
   tatttgtgcc gcaattgccc atacgtttcg tggaatgttt cttcactgtg gcgacatttc 840
   cgtcatcata ttcaaaaatc gaaacaatct tggacgtgta tcgcatgctc atacagctca 900
   tcgagccggg tgaaaattga tctacatgtg aaaatgcata aggaaatgcc ggaaattgat 960
   ttggagtttg caacttggct tcgctatgaa cgaagaatca acaagaacga cttgaataaa 1020
   ccgactaaca aaaaaaagaa acccgatgga ggaaacggtt caaatcacag tgatatgaga 1080
   agtcttcacg catttcttag tcttaaaaat tctaagaaca atgttgtcaa acacgatatc 1140
   gatgctccaa ctttgcaccc attatcccca gcgcccaagc tggtcgcgat gactcaattt 1200
   gacttcggag aaattgtcac ttacaaaagc gtcaatccgc tgcaccaaat caacaagaac 1260
   aatagcaatc cgacggtcct gccgaacaag cgaaatagca ttaaaaccaa tcaattccaa 1320
   caatccgtga aagttccaaa gtacgaagac tttttgaaca tgaagccagt tatgccatat 1380
   ttccaaaaac aacgacatcc actggaagcg attgctatgt acgaaaaggc aaaaagggaa 1440
   tacgaaaaga atcactgttt cccgaatctt ccaattttcg aattcaacat tgagtacaag 1500
   aacttgcatc cactcgccaa ggcacagtat gggaaaaaca atatgaaaga gtattttttg 1560
   aatgaaatgg aagtagagaa atctcgcgaa tgcactgact gcccattcaa acacaacgat 1620
   ctgcagcagt tccgtctcca ccgtgacaag catttctacg gtggatcaca cacctgtccg 1680
   gagtgcaact attcatcgaa caaccataat caagtggtcg agcacacgtt tgtggatcac 1740
   tatttgagcg atgtgagatt ggttgaaggg cttccgtctt cggattcgga ggacgataat 1800
   attccggtac ctcctgatac tccgcagcgg aagaaaaaag cgccgaagcg tggcaaacgt 1860
   cgtggctggt aaagattata ttttattaaa ttttatcaag tttttttaaa ggtaaatgtt 1920
   taaatttatt ttaaatttaa atttattttt gtttcttttt ttttcaatta attgttttta 1980
   agtttttcaa tgttaggttt gtcgtttaat ttttttcaaa tttctaataa tattcccttc 2040
   ccttcccatc cccgctcaaa tagctgcctc atagtttagt aaatttctaa ataatattat 2100
   ttcaatacca tcacatattg tcagcgcttt ctgtcatttt tttaattttc attcatcctg 2160
   gtacctcact tttggcacgt gtccaacaac caccctgaca tcaacccacc cggactttct 2220
   cgactaggtt tttttgaaat tcttccccca ttttattttc tttgaccatt ttatgtttgt 2280
   gttttgaatg aaatgtgttt tcgaattttt aaaa 2314
<210> 7
   <211> 9260
   <212> DNA
   <213> Caenorhabditis elegans
<220>
   <223> Sequenz von pBy1015, dieser Abschnitt komplementiert den Defekt der ses-1 Nullmutante
<400> 7
   gatccagcaa aacttgtcaa ctctgacttg tctgtttcca tcaaagaagg ttccaattac 60
   cggttaagct ttgctttcca tgtgcaacgc gaaattacgt ccggattgca gttagttttt 120
   ttgtgtgttt aatcttaact taaaataata aattgtttca gctacaaaca taaggtgaaa 180
   cgatccggta tcactgtgga gaatgagaag tacatgatgg gaagctacgc gccaaaactt 240
   gagattcagg agtacaaatc accaaacgaa gaagctccgt ctggaatgat gcatcgtgga 300
   aagtacaagg tgtactcgaa aattaccgac gatgacaaca atgtgtatct tgactggcaa 360
   tggacccttc acattaccaa ggagtagaat aattttccgc ctttactgaa cctcatattt 420
   atattcaaat tctcaatgaa aatgactttt ttctccaatt taccgcccac cccaaatgtt 480
   ttccttgttt tatggttatt attgattttg atgcactagt tgtgttgtgt gatgcctctc 540
   ccctttcccc gccaaattga aatgatatat aaagtgatat ttcataatca tttaaaacaa 600
   agtgtactgt tttgtgaaac gttggaaact aaccaaaaaa cactaacttg ctgcgtttcc 660
   aaaaacttaa gatcctaata cgcatttaca cgttgttccg caatattcac gtggtttcag 720
   ctgggccgga acattgtatt aatttcagaa ttagagattt tattttgata caataagaat 780
   tgaactacaa aacgttacaa ctttgaaaat ttaaaagagt actaacggtt tggaagtttt 840
   gaacgtataa acccaaaatg tgttaaaata aattaatttc gtaataaaaa gtcaaaaaaa 900
   tttgaataga cttttctcac aaaaatccaa acttaaaaaa aaatttacca gaaactttgt 960
   agcaaatttt cagatctttt tactgaatac ctaagtattt ttggtactcg tgtgttacct 1020
   aaaattactc tgtagttcta actttcaaaa ttactatgta gttctaactt ttaaagttac 1080
   tttaagatta gtaaattgaa ttcgtgcaaa atgtttgaaa attgattttc aagacattaa 1140
   atgatgatca ccattacaaa aatctttgca aaaacttact gccgtaaatt aaaaagaatt 1200
   tatcgaatca caccactacg tgtgggtgta gtctgaagaa attcatgctt tcttgctcca 1260
   ttaggaaata cttaatgaaa ttggaaattt tatcggtgct gggccagaag agccaacaag 1320
   atccattttg aatttcctca acttggaatt tgaatttgat aatatgagct cgttgctcca 1380
   cctcatgctt ctttttagtt ttttgaatat tgtgacaact agtcgcgttg agtgcatttg 1440
   agtgcattgt ggttctggaa tgtgatccac taaccagctg ctattccttg aaaggtaagt 1500
   tacaatagtt tttctttact ttacaaatcg ataaagtcaa tcattgactc aactttcgat 1560
   ctgagaaatt tctttcaaat atttcaaggt gcgctgattt cagctgtcaa attattttat 1620
   aaccaaaagt ttttgacatg atctcccata agttttctaa caacctttgg atatgttata 1680
   tcattaaata tattcattaa tatcaaagtt gaaaatttta ttggtttatg aaattcactg 1740
   aaaaatgcta tcaaatgtga gcatttatgg ggttattcaa gtaatcttgc ttcatcagtt 1800
   atgggacatt cgtaaatagt ctcgctattg gtcttgcacg catacggatc atttcaaaat 1860
   tagtcttgaa tgcaagccta acagaggaaa tacgttattc atttttcagg aggcttccca 1920
   tttgtttatg gtaacaaaaa ggaagaagac tggcgtgcag gaggaaaacg atacagtatg 1980
   tttttggcaa gaaacgtgga gactgtccgt tgtgttttat acgataattc cgagttctga 2040
   actagactta acgtcaacag acattctaaa aaactattcc ttacattgaa aatgaagagg 2100
   ttttctgttc taaatgattt tattgtaata aatatttctt atgtttctta atcttatgta 2160
   gacatgaaag ctgtttagtt gttcttttga aaagaaatta agatgaaatg aaagagaaag 2220
   gaagcaacgg gtttttcgat ttttcttcaa aatcagctgt tgacatttct tattatatgg 2280
   ttaaaattga tatttctatt gaaaaaaaac atcaagtttg aaatcatttt cgaaaactga 2340
   agttcaaatt tctacgcctc ttgtgttcat cttaacctaa attaaagctt agcttaactc 2400
   aaataagact tttcaaagtc tgaaattatt aagtctagat acacctcccc ctccccctca 2460
   ctgttctttt ttactttcaa tcgatcaata tctgaacagt atacaactac gctctccact 2520
   ctcattagaa aataacagtg taccttttcg atgcggtacg cgtcgcggca ccaacgcggc 2580
   acacacgcag cgcagtccca tctacttttt ctcggtggtt cattgcacac cgatcctcac 2640
   ccagcacaca gttttctctc cttaacagtt ggggctgaca ttgctaactc ttccctccgc 2700
   tcacctgttt attctccttt ttctagttgt ctcgcggtct gcgtcttctc attttatata 2760
   catttagagt ttagggtgag aacccaattt ttcatggtcc atttttgttg gctaaattct 2820
   ttacatgttg aatattttaa atgctaccac atttggactt actctagaat aagttatctt 2880
   aagacgagtt attttagact agactaacca gttaggaata ttttatcaat ttccgatttt 2940
   gaaacccaaa aaatcaatgt taatttagca ctgaaacatg ctggtaggca ctcaatattt 3000
   taagcttcta gctagaacac ttaggacttt acttacggaa tagcctttta gacttttagt 3060
   tagccgtttg gggttaggaa aaacaaagaa ccctagaagc tatgtttccg ttgagaacca 3120
   atgaccatct atctttcaac gaacatttca cctaataaca gagtcatgag attgtgactt 3180
   gtaaatattt ttttccatct ttcccctgtt tcagtcgtca tttgaacgtt ctctttcttt 3240
   tcttcctcga cagtgaaaat aataaacgta aaaggtttta aaatcaccca ttttcaaatt 3300
   ccatcgtgta atttcttatc acaatagtac ccacaatcgc ttctcgtttc ccagcagtta 3360
   tcttaaagca ttagccaaca tctgttgtcc ggccgttctc atctcttccg ctgtctacgt 3420
   ctctatcctt cgtctcacta attcaccctt cttttctctc tccgtagggc tagggtgcgc 3480
   gcctcgcggc cagtagaact gtgttgtaga tgcgaaaaga gtgcggtcta cttctaagcg 3540
   cagacatatt gtcaacgggg gctgcggacg acgcgggagc cgccagtttt ggaagtgtag 3600
   tactcgcatt cccccccccc cccccccccc ccacatgcgc ttcgttttcc cttgacttcc 3660
   attatcactg tcaattctct tgtcaactgc catttgtata aatcatcaat cctatgttaa 3720
   tattatttca gtagaagacg atgccacctc gcaagcgcaa acttgaggag ttgaagaatg 3780
   aacaaggaga ggtaggacag cgttgagatt ttttttcaaa aacttaatga tttaatttta 3840
   gcaggggtca tacaaatgcc atctttgtgg acagtgcttc taccgtggat gtggattagc 3900
   aagtcacctg cgacgacacg caccagtcac atttgactgt gagcattgtg cttacacgtg 3960
   taagcacaaa tatgcgtatg atcgtcatct gttgcaatcg catcctgagc tcgtggaatc 4020
   tggcccacta gttcgaatgt ttaagaatga agatgaagat gctccaatgc cgattctcga 4080
   gcgagaagca gatgtatccg atgatgccgt caagtatgag gcactctctt ctttctcgga 4140
   acctgaacca atcagctaca aatgtccatt gtgtatatcc acgtttggca gtcacgctag 4200
   agcagtctac catattttgt cacaccgtgt caagttgtat caagcaccga aaagcctgaa 4260
   attcttctcg aggaaaacaa tgcaagctct tggaggattt cgtttagagt cgcaactgat 4320
   gatcaaatgg agattacaat acgataatcg aagtgtggac gagaagcgaa cacggttatg 4380
   gcggtatatg gaagaaaatt ttggacatga acatctgaag caaccaaagc tggaaagtga 4440
   taatccttcg acaagtagct cttttgagtg agttttttgc ccaacgagct tttgatagaa 4500
   aaaaataatg ttagaaacca tgagtcactt tttcatttca aaattaatat acaatactaa 4560
   ccacatcttt cagatcacaa acaaacttga acgattcttc gataatcaaa aagaaacttg 4620
   tgatgaagtg tggtactgtt ttcggtcaaa gactcattca cgacaacacc caatattatt 4680
   tgtgccgcaa ttgcccatac gtttcgtgga atgtttcttc actgtggcga catttccgtc 4740
   atcatattca aaaatcgaaa caatcttgga cgtgtatcgc atgctcatac agctcatcga 4800
   gccgggtgaa aattgatcta catgtgaaaa tgcataagga aatgccggaa attgatttgg 4860
   aggtaagaaa tatatcatca taaccaaaat taaataattt tatttaaaaa aaaaatttca 4920
   gtttgcaact tggcttcgct atgaacgaag aatcaacaag aacgacttga ataaaccgac 4980
   taacaaaaaa aagaaacccg atggaggaaa cggttcaaat cacagtgata tgagaagtct 5040
   tcacgcattt cttagtctta aaaattctaa gaacaatgtt gtcaaacacg atatcgatgc 5100
   tccaactttg cacccattat ccccagcgcc caagctggtc gcgatgactc aatttgactt 5160
   cggagaaatt gtcacttaca aaagcgtcaa tccgctgcac caaatcaaca agaacaatag 5220
   caatccgacg gtcctgccga acaagcgaaa tagcattaaa accagtaaaa gtgatactca 5280
   aatcgcgctc agcgttaagc agtctagtag tatgaagatg gtgaaagttt ctcctggaaa 5340
   agtttatcag cttcctaaga cgtccaaatt ttaccgacct gaatcacctg actcattagc 5400
   atcaaataat tctgctcacg gagacgagat tgagtcaacg tcttcagatc aattccaaca 5460
   atccgtgaaa gttccaaagt acgaagactt tttgaacatg aagccagtta tgccatattt 5520
   ccaaaaacaa cgacatccac tggaagcgat tgctatgtac gaaaaggcaa aaagggaata 5580
   cgaaaagaat cactgtttcc cgaatcttcc aattttcgaa ttcaacattg agtacaagaa 5640
   cttgcatcca ctcgccaagg cacagtatgg gaaaaacaat atgaaagagt attttttgaa 5700
   tgaaatggaa gtagagagta agttaacgag tcttcaacga agtacatatt ctgaaaaaaa 5760
   ctagaacatt tttatcaaat ggctcttttt cagaatctcg cgaatgcact gactgcccat 5820
   tcaaacacaa cgatctgcag cagttccgtc tccaccgtga caagcatttc tacggtggat 5880
   cacacacctg tccggagtgc aactattcat cgaacaacca taatcaagtg gtcgagcaca 5940
   cgtttgtgga tcactatttg agcgatgtga gattggttga agggcttccg tcttcggatt 6000
   cggaggacga taatattccg gtacctcctg atactccgca gcggaagaaa aaagcgccga 6060
   agcgtggcaa acgtcgtggc tggtaaagat tatattttat taaattttat caagtttttt 6120
   taaaggtaat gtttaaattt attttaaatt taaatttatt tttgtttctt tttttttcaa 6180
   ttaattgttt ttaagttttt caatgttagg tttgtcgttt aatttttttc aaatttctaa 6240
   taatattccc ttcccttccc atccccgctc aaatagctgc ctcatagttt agtaaatttc 6300
   taaataatat tatttcaata ccatcacata ttgtcagcgc tttctgtcat ttttttaatt 6360
   ttcattcatc ctggtacctc acttttggca cgtgtccaac aaccaccctg acatcaaccc 6420
   acccggactt tctcgactag gtttttttga aattcttccc ccattttatt ttctttgacc 6480
   attttatgtt tgtgttttga atgaaatgtg ttttcgaatt tttaaaattt gttttagact 6540
   ttggaatctg ggtaaaaata tattactgaa tgtcgtcagt tttgatgctt gcacagccaa 6600
   ctgcagatac acaaaaggca aaattgcgga cttaggagtg ttggatatac ggaaatgaac 6660
   atacgtgtta atcagtttaa cggattgcaa attcaatggg ttttcaaatt ttcagatata 6720
   ttatatgtag tatcgaagca attaaggtaa aagcgataga tataaacatt atgaagcctg 6780
   tatgaaattg gcttctatta aatatttaca ttaatcgaat aacattttac aacttgcaac 6840
   gtatgactca atcttttata aacgccagaa aaccatttaa aacggaagca ccgattttaa 6900
   cactaatttt tcaaacaagc cggatcagac taacacggac taaacttaga catagataaa 6960
   gtgttgaaaa agaacctata ttgacatgtg agtagctagg actggtctga atacatcaga 7020
   acaggaaaaa ataacagaat atctcttacc aaaactataa atagaaaaaa aagttgatca 7080
   aggctgtagc agttcagagt aaagccaaag taagatattg ataaggagag tttctcagag 7140
   taaaaccatg tggattagca aaaaatgctg aaactatttc gtcgagccat tccaattcag 7200
   aaatcttact gtcagatttt tcgacacttt ttgactgtcg catatccaag acgttcctgt 7260
   ttttgaatta taccgaagat tttcggaatt catttggctt tttctctgac ttttcatctt 7320
   ttatggaaaa tctagtaatt ctggcttctt ccaattcttt acagtttgtt tgaattttct 7380
   ggatacgaag cattttatta cacaggtaca gaaatggtta tctcgaataa atataacact 7440
   ttagtacgga tagggaatga aattgacatt attcacaacg attttcagca tcttttgagt 7500
   agttggctag ccagctgttt tgatttacaa tttacaattt gtcgttttcc tcttgctcct 7560
   ttgtttctga aaaatgtata atttcatata catataaatt cagaaaattt gttgaaaata 7620
   caacgtttac tcacttttct cactctgatt ctcaagccac ttctccattt cagcagcttc 7680
   gtgctcggtt ggcaacttgt tgctcacagc ggctgcaagt ccaacgtcgg tttgtggctc 7740
   agcgtttgcc ttgtaagcat cctgagaggt tgaggcgctg cttgatggct ctccgctggc 7800
   agcagttacc tctgaaatta gataattcaa ttttgttttt cttttcgtaa cttactgagg 7860
   gctgataatt ggtcacccaa actttttccg tcacccatat caattagatc acgagcttcg 7920
   gcggcttgct tttccccgtc gcggtttgac acaaaacgat catatttttc aaaaacactg 7980
   ttcaatgaat cattgaccat aagaagttca tctaaaacac aagattttag tttcagtcgc 8040
   aaaaaacacc cattcttaca agtaacttca tcattgctca cgtagcggat caggtccagc 8100
   acgcgttgtt gcatgtgacg gcaagtgtcg ttgaggtccg atagcaattg cagctcgtcg 8160
   gcggtctcct tgcgtggcac aacgtcggtg agcgtctcgc ggaacacttt gatgttttga 8220
   ttgaccacgt caaggtctgc ccgcaatttg gtcaattgcg ctggtgttgc ggagattggc 8280
   tcttgtccct gctcgcggat ggtgagaacg tcgtatgtct aaaatgtagt aattgttacg 8340
   tgtttttgga ataaaaaaac aaacaaactt tgtaatgaaa cttttcaaaa tttttcagaa 8400
   atgtttcatg acggttcaaa attttcaaca aaaaaaaaac gctaagtcta gctaaaactc 8460
   caattttgat tacttttccg taccgcagca gctggaaaac aatttttgtt gaattatcat 8520
   cccttatcga atttttggta gtaatttttg aacattccgt tgactaaggt acttttaaag 8580
   ccgataaggc aatttttggt tagatatgac tttggatgta ttttaatcga cgaaccaacg 8640
   taagataaac ccactaaata ttacaacaag attgcttgaa aaatgctatg atacacctgc 8700
   tgatgtataa aaaaaacagg ccaaaaatgg atattttagc tagaagtagc gttttttttt 8760
   tgaaaatttt gaacaatcat aaataatttg tgaaacattt tttaaaaatc tcataacgaa 8820
   ataagctcat ttaagcacat cttatgtcta tacattgaca taaacgcttt tcgttagccc 8880
   tcctatacaa aactctattc atatttgtta ttgactagtg acgctgacgc tttcacaact 8940
   ttgaataata ataataaagc ttgaaatcac aaaacaatat ctaccgattg ggggttttgt 9000
   tggccttgct gtggttgttg ttgctgttgc tgattttgtt gttgttgatc atctagggtt 9060
   gccggtggag gttgattaaa tactgtctga attcacaaca ctatatgtat gcgcttcaca 9120
   acaatgatct tcacaagtat acaaaatact cactcgtttt ggggttttaa ttggagccag 9180
   agtatcaaga tcagccgccg ggaattcgac tcccttactc ttcaagtcat cgtatgattg 9240
   tacaactccg gcaagtgtag                                             9260

## Beschreibung

Die vorliegende Erfindung betrifft isolierte Nukleinsäuremoleküle, die für *ses-1* bzw. mutierte *ses-1* codieren, Vektoren und transgene Organismen, welche solche Nukleinsäuremoleküle enthalten, Verwendungen solcher Nukleinsäuremoleküle zur Herstellung von Arzneimitteln sowie zur Erzeugung von nichtmenschlichen Modellorganismen.

### Hintergrund der Erfindung

Die Alzheimer'sche Krankheit ist neben der Parkinsonschen Krankheit die bekannteste neurodegenerative Erkrankung. Das charakteristische Merkmal der Alzheimer'schen Erkrankung ist die Entwicklung neuronaler Proteinaggregationen, sogenannter Plaques, die im wesentlichen aus einem unlöslichen, 4 kDa großen Peptid namens Amyloid Beta-Peptide (Aß4) bestehen. Untersuchungen der letzten Jahre deuten darauf hin, dass die Bildung von Aβ4 ursächlich an der Entstehung der Krankheit beteiligt ist. Dominante Mutationen in drei Genen verursachen familiäre Formen der Erkrankung (FAD Familial Alzheimer's Disease). Es wurde gefunden, dass eines der betroffenen Gene für das Amyloid Precursor Protein (APP) kodiert, das durch drei Proteasen prozessiert werden kann, wobei unter anderem Aβ4 entsteht. FAD Mutationen in APP erhöhen die Produktionsmenge von Aß42, einer 42 Aminosäure langen Variante von Aβ4.

Molekulargenetische Untersuchungen von Familien, in denen die Alzheimer'sche Krankheit auftrat, haben zu der Identifizierung der humanen Gene Presenilin 1 und Presenilin 2 geführt (Rogaev et al. 1995, Nature 376, 775-778; Levy-Lahad, E. et al. 1995, Science 269: 973-977), die, durch bestimmte Mutationen verändert, ursächlich am Ausbruch der Alzheimer'schen Krankheit beteiligt sind und welche außerdem während der Entwicklung eine Schlüsselrolle in dem Notch-Signaltransduktionsweg spielen. Presenilinproteine sind ER-Golgi lokalisiert und besitzen mindestens 6 Transmembrandomänen. Es wurde gefunden, dass Mutationen in PS1 und PS2 die Bildung von Aβ4 beeinflussen und an der Verursachung der Alzheimer'schen Erkrankung beteiligt sind, vermutlich über die Entstehung von Amyloidablagerungen. Mitglieder der Presenilinfamilie wurden unter anderem in Maus, *Drosophila melanogaster, Xenopus laevis* und im Fadenwurm *Caenorhabditis elegans* identifiziert. Obwohl mutierte Preseniline die Prozessierung von APP im Menschen beeinflussen, ist ihre natürliche Funktion dort nicht detailliert bekannt; eine Funktion als APP-Protease (gamma-Sekretase) wurde postuliert. Aus weiteren Untersuchungen mit menschlichen Zellkulturen ergaben sich Hinweise darauf, dass Preseniline bei der intrazellulären Proteolyese von Notch-ähnlichen Rezeptoren nach deren Aktivierung durch Ligandenbindung eine Rolle spielen. Daher wird angenommen, dass die Preseniline bei der Prozessierung von mindestens zwei unterschiedlichen Membranproteinen (APP und Notch) beteiligt sind. Es wird gegenwärtig kontrovers diskutiert, ob die Preseniline selbst die Proteasen in dieser Prozessierung sind, oder ob sie Kofaktoren dieser Reaktionen sind oder die Proteolyse indirekt beeinflussen (Haass, C. et al. 1999, Science 286 (5441): 916-919).

Ausgehend von den erwähnten Presenilin-Genen wird intensiv geforscht, um die molekularen Erkrankungsprozesse aufzuklären und um Arzneimittel für die Behandlung und Prävention der Alzheimer'schen Krankheit zu entwickeln. Insbesondere wird dabei nach Faktoren gesucht, die die Aktivität der Presenilin-Gene beeinflussen. Dabei kommt der Suche nach Suppressoren des Presenilin-Mutanten Phänotyps eine besondere Bedeutung zu. Suppressoren können Mutationen in anderen Genen sein, die die Notwendigkeit der Presenilinfunktion eliminieren, oder es können Substanzen sein, die die Aktivität anderer Gene oder der Preseniline modulieren, so dass der Presenilin-Mutanten-Defekt keine phänotypischen Auswirkungen hat.

Als ein bevorzugter Modellorganismus wurde bei solchen Untersuchungen vielfach ein Nematode, insbesondere *Caenorhabditis elegans* eingesetzt. Der Vorteil eines Nematodenmodells, insbesondere des *C. elegans*-Modells, besteht vor allem in der Eignung für ein Hochdurchsatzverfahren (HTS; High-Throughput-Screening), der Möglichkeit einer schnelleren genetischen Analyse durch geringere Generationszeit (2-3 Tage) und einem detaillierterem Wissen über molekulare und funktionelle Eigenschaften des Nervensystems in *C. elegans.*
Da *C*. *elegans* in Mikrotiterplatten gehalten werden kann, können mit diesem Testsystem 10.000 und mehr Substanzen in kurzer Zeit per HTS am lebenden Wurm ausgetestet werden.

Im Fadenwurm C. elegans wurden bisher drei Presenilingene namens *sel-12, hop-1* und *spe-4* identifiziert. *Spe-4* ist das divergenteste Mitglied der Familie. Mutationen in *spe-4* führen zu einem Defekt der cytoplasmatischen Partitionierung während der Spermatogenese (L'Hernault, S. W. et al. 1992, J Cell Biol 119: 55-68). Mutationen in *hop-1* haben keinen sichtbaren Phänotyp, führen aber in Kombination mit Mutationen in *sel-12* zu einem synthetisch letalen Phänotyp (Li, X. et al. 1997, Proc Natl Acad Sci USA 94: 12204-12209). *Sel-12* ähnelt den menschlichen PS1 und PS2 am stärksten und ist auch das am besten studierte Presenilingen in *C. elegans.* Es wurde gefunden, daß bestimmte *sel-12* Mutanten einen Eiablagedefekt aufweisen und morphologische Veränderungen der Vulva-Entwicklung zeigen (Levitan, D. et al. 1995, Nature 377: 351-4). *Sel-12* ist 50 % identisch zu humanen Presenilinen und der Eiablagedefekt von *sel-12* Mutanten lässt sich durch transgene Expression von PS1 oder PS2 vom *sel-12* Promoter aus kompensieren (Baumeister et al., 1997, Genes and Function 1: 149-159). Dies zeigt, dass PS1 und PS2 funktionelle Homologie zu *sel-12* aufweisen. *Sel-12* Mutationen wurden zuerst als Suppressoren des Multivulva Phänotyps einer Funktionsgewinnmutante (gain-of-function) im *lin-12* und *glp-1* Notch Rezeptor-Gen identifiziert. Notch Rezeptoren kontrollieren die Determinierung von Zellschicksalen in vielen mehrzelligen Organismen. Außerdem wurde gezeigt, dass der Phänotyp von *sel-12* Mutanten dem von schwachen Funktionsverlust-Mutanten von *lin-12* ähnelt. Zusammenfassend lässt sich feststellen, dass Preseniline bei der Notch Signaltransduktion in zahlreichen Organismen, einschließlich den Säugern, eine wichtige Rolle spielen.

### Zusammenfassung der Erfindung

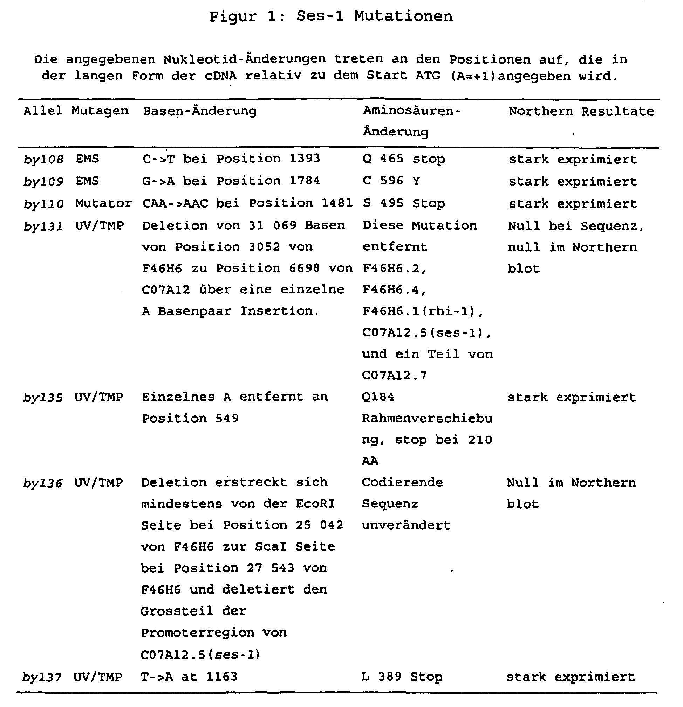

Die gegenwärtigen Erfinder haben nun überraschend festgestellt, dass bestimmte Mutationen, wie z.B. in Figur 1 aufgeführt, in einem weiteren Gen, dem von den Erfindern erstmals identifizierten *ses-1*-Gen von *C. elegans,* einen Defekt, insbesondere den Eiablagedefekt einer *sel-12*-Mutante unterdrücken können. Das heißt, bestimmte Mutationen in dem *ses-1*-Gen führen dazu, dass z.B. der Eiablagedefekt, der durch bestimmte *sel-12-*Mutationen verursacht wird, behoben wird und die Doppelmutanten (*sel-12⁻; ses-1*⁻) wieder einen normalen Wildtyp-Phänotyp zeigen. Genauere Untersuchungen ergaben, daß diese Mutationen in dem *ses-1*-Gen bei den *sel-12*-Mutanten zu einer Aktivierung von *hop-1* führen, d.h. das *hop-1* Presenilin-Protein übernimmt die Funktion des defekten *sel-12* Presenilin-Proteins.

Die gegenwärtigen Erfinder haben das neue *ses-1*-Gen auf dem Genom von *C*. *elegans* lokalisiert und sequenziert.

Somit betrifft die vorliegende Erfindung zunächst ein isoliertes Nukleinsäuremolekül, das für SES-1 Protein codiert, sowie weitere Nukleinsäuremoleküle, die für mutierte SES-1 Proteine codieren.

Darüber hinaus betrifft die Erfindung einen Vektor, der solche isolierten Nukleinsäuremoleküle oder Fragmente davon umfasst.

Weiterhin betrifft die Erfindung die von den Nukleinsäuremolekülen codierten SES-1 Proteine und mutierte SES-1-Proteine oder Fragmente davon sowie Antikörper, die mit Hilfe dieser Proteine erzeugt wurden.

Außerdem betrifft die Erfindung einen transgenen nichtmenschlichen Organismus, welcher ein isoliertes, für SES-1-Protein oder mutierte SES-1 Protein codierendes Nukleinsäuremolekül umfasst. Insbesondere beschreibt die Erfindung transgene *C. elegans,* die ein *ses-1* Allel aufweisen, das die Aktivität von *ses-1* vermindert resp. die Aktivität von *sel-12* oder *hop-1* steigert. In diesem Zusammenhang werden unter transgenen Organismen auch solche Organismen verstanden, bei denen das Genom durch Mutation verändert wurde, und die dadurch ein mutiertes ses-1 Gen aufweisen, oder denen das *ses-1* Gen entfernt worden ist.

Schließlich betrifft die Erfindung Verwendungen solcher Nukleinsäuremoleküle zur Herstellung eines Arzneimittels gegen die Alzheimer'sche Krankheit sowie zur Erzeugung von nichtmenschlichen Modellsystemen zur weiteren Erforschung dieser Erkrankung.

Insbesondere beschreibt die Erfindung die Verwendung von transgenen *C. elegans* in einem Verfahren zur Identifizierung und/oder Charakterisierung von Substanzen, die die Aktivität von *ses-1* vermindern resp. die Aktivität von *sel-12* oder *hop-1* steigern.

Zudem beschreibt die Erfindung auch die Verwendung von Substanzen, die die Expression des humanen Presenilin 1 oder 2 steigern, zur Behandlung der Alzheimer' schen Krankheit, sowie diese Substanzen selbst und pharmazeutische Zusammensetzungen, die diese Substanzen enthalten.

### Detaillierte Beschreibung der Erfindung

Die vorliegende Erfindung basiert darauf, dass ein neues Gen, *ses-1,* in *C. elegans* identifiziert werden konnte, welches mit den *C. elegans-*Presenilin-Genen *sel-12* und *hop-1* wechselwirkt und damit eine wichtige Rolle bei der Entwicklung der Alzheimer'schen Krankheit spielt.

So führten verschiedene Mutationen in dem *ses-1-*Gen, darunter auch die vollständige Deletion des *ses-1-*Gens, dazu, dass bestimmte von *sel-12*-Mutationen hervorgerufene veränderte Phänotypen supprimiert bzw. unterdrückt wurden. Die Suppression solcher *sel-12*-Defekte in *ses-1*-Mutanten wird dadurch erreicht, dass sie die Expression von *hop-1* deutlich dereprimieren und das HOP-1 Protein vermutlich die Funktion des defekten *sel-12-*Proteins übernehmen kann.

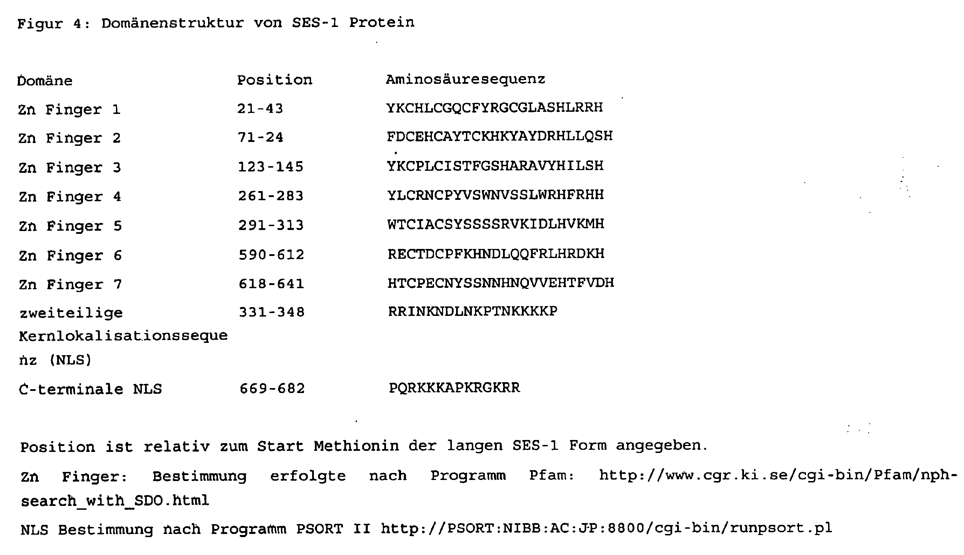

Das identifizierte *ses-1-*Gen enthielt bis zu 7 vorhergesagte C2H2 Zinkfingerdomänen und zwei Bereiche, die als Kernlokalisationssignale (Figur 4) dienen könnten.

Das abgeleitete SES-1 Protein zeigte bei einem Sequenzvergleich in der Datenbank Genbank mit Hilfe des BLAST-Programms keine deutliche Ähnlichkeit mit irgendeinem bekannten Protein. Es wurde lediglich eine schwache Ähnlichkeit mit verschiedenen Proteinen festgestellt, die ebenfalls Zinkfingerdomänen enthielten.

Somit stellt die vorliegende Erfindung ein isoliertes Nukleinsäuremolekül zur Verfügung, das für SES-1-Protein codiert.

Weiterhin umfasst die Erfindung isolierte Nukleinsäuremoleküle, die für mutierte SES-1 Proteine codieren. Insbesondere werden isolierte Nukleinsäuremoleküle zur Verfügung gestellt, die für mutierte SES-1 Proteine codieren, die wenigstens eine der in Figur 1 angegebenen Mutationen aufweisen.

Darüber hinaus betreffen weitere Aspekte der Erfindung isolierte Nukleinsäuremoleküle, insbesondere DNA-, wie cDNA- oder genomische DNA-Moleküle, aber auch z.B. RNA-Moleküle, die ein SES-1 Protein aus *C. elegans* mit der in SEQ ID NR:1 oder SEQ ID NR:2 angegebenen Aminosäuresequenz codieren, insbesondere jene, die eine in SEQ ID NR:3, SEQ ID NR:4, SEQ ID NR:5 oder SEQ ID NR:6 angegebene Nukleinsäuresequenz aufweisen.

Die erfindungsgemäßen Nukleinsäuremoleküle mit Nukleinsäuresequenzähnlichkeit zu der in SEQ ID NR:3, SEQ ID NR:4, SEQ ID NR:5 oder SEQ ID NR:6 angegebenen Nukleinsäuresequenz umfassen insbesondere auch allelische Varianten der angegebenen Nukleinsäuresequenz, die insbesondere die oben erwähnten Mutationen einschließen.

Die Erfindung beschreibt auch Nukleinsäuremoleküle mit jeweils zu den oben erläuterten Nukleinsäuresequenzen komplementärer Nukleinsäuresequenz.

Der Begriff "isoliertes Nukleinsäuremolekül" bezieht sich im vorliegenden Zusammenhang auf Nukleinsäuremoleküle, die im wesentlichen gereinigt von der Hauptmenge der andersartigen Nukleinsäuremoleküle aus den Ausgangs- bzw. Produzentenzellen vorliegen. Präparationen von erfindungsgemäßen isolierten Nukleinsäuremolekülen können jedoch ohne weiteres weitere Bestandteile, wie Salze, Substanzen aus dem Medium oder auch restliche Bestandteile der Produzentenzellen, wie z.B. diverse Proteine, enthalten.

Die Erfindung umfasst ebenso Vektoren, welche ein erfindungsgemäßes Nukleinsäuremolekül, das für SES-1 Protein oder mutierte SES-1 Proteine codiert, enthalten. Beschrieben sind auch Vektoren, die ein Fragment eines erfindungsgemäßen Nukleinsäuremoleküls aufweisen. Das Nukleinsäuremolekül ist dabei mit einem Promoter verknüpft, der für eine Expression des Nukleinsäuremoleküls in dem zur Expression verwendeten Organismus sorgt. Für eine Expression in *C*. *elegans* wird dabei insbesondere einer der bekannten *C*. *elegans*-Promotoren (http://chinook.uoregon.edu/promoters.html) verwendet.

Der Vektor kann beispielsweise ein Plasmid, ein Cosmid, ein Bacmid, ein Virus oder ein Bakteriophage sein.

Ein weiterer Aspekt betrifft die Polypeptide oder Proteine, die von den erläuterten Nukleinsäuremolekülen codiert werden, insbesondere SES-1 Protein aus *C. elegans* mit der in SEQ ID NR:1 oder SEQ ID NR:2 angegebenen Aminosäuresequenz und die oben erwähnten mutierten SES-1 Proteine. Gleichfalls umfasst sind Fusionsproteine, die erfindungsgemäße Polypeptide, Proteine oder Proteinfragmente fusioniert an ein andersartiges Protein oder einen andersartigen Proteinabschnitt, z.B. ein Marker- oder Indikatorprotein, enthalten. Beschrieben sind auch Fragmente der oben erwähnten Proteine oder Polypeptide.

Die mit Hilfe solcher Proteine erhältlichen Antikörper, welche monoklonal oder polyklonal sein können, sind von der Erfindung ebenfalls umfasst.

Die Erfindung erstreckt sich auch auf transgener nichtmenschliche Organismen, insbesondere Mikroorganismen, z.B. Bakterien, Viren, Protozoen, Pilze und Hefen, Algen, Pflanzen oder Tiere, wie auch Teile, z.B. Zellen, und Vermehrungsmaterial, z.B. Samen, von solchen transgenen Organismen, die eine rekombinante Nukleinsäuresequenz - ggf. in ein Chromosom integriert oder auch extrachromosomal - umfassen, die ein erfindungsgemäßes Nukleinsäuremolekül, das für SES-1 Protein oder mutiertes SES-1 Protein davon codiert, als Transgen enthält. Unter einem bevorzugten Aspekt werden die transgenen nichtmenschlichen Organismen das von dem erwähnten Transgen codierte Polypeptid oder Protein, d.h. SES-1 Protein aus *C. elegans* oder ein davon abgeleitetes Polypeptid oder Protein, auch exprimieren.

Gemäß einem Aspekt weisen transgene C. *elegans* ein *ses-1* Allel auf, das die Aktivität von *ses-1* vermindert, verstärkt oder ausschaltet. Teil der Erfindung sind auch solche transgenen *C. elegans,* denen das *ses-1-*Gen vollständig entfernt worden ist. Insbesondere betrifft die vorliegende Erfindung transgene *C. elegans,* die ein *ses-1* Allel aufweisen, das den Eiablagedefekt Phänotyp (Egl) in *sel-12*-Mutanten kompensiert. Zudem betrifft die vorliegende Erfindung transgene *C. elegans,* die ein *ses-1* Allel aufweisen, das die Aktivität von *hop-1* oder *sel-12* steigert.

Die Erfindung erfasst auch die Verwendung des erfindungsgemäßen Nukleinsäuremoleküls zur Herstellung eines Arzneimittels zur Behandlung neurologischer, insbesondere neurodegenerativer Erkrankungen, besonders bevorzugt der Alzheimer' schen Krankheit bzw. zur Erzeugung eines nichtmenschlichen Modellorganismus zur weiteren Untersuchung und Erforschung neurologischer, insbesondere neurodegenerativer Erkrankungen.

Die oben erwähnten Arzneimittel sind beispielsweise dadurch erhältlich, dass ein erfindungsgemäßes Nukleinsäuremolekül selbst als Gentherapeutikum eingesetzt wird oder indem mit Hilfe eines solchen Nukleinsäuremoleküls ein Modellorganismus aufgebaut wird und die mit Hilfe dieses Modellorganismus aufgefundenen Substanzen zu einem Arzneimittel formuliert werden. Für nähere Einzelheiten hierzu wird z.B. auf die internationale Anmeldung PCT/EP01/03214 desselben Anmelders verwiesen.

Insbesondere beschreibt die Erfindung die Verwendung von transgenen C. *elegans,* die ein erfindungsgemäßes Nukleinsäuremolekül aufweisen, in einem Verfahren zur Identifizierung und/oder Charakterisierung von Substanzen, die die *ses-1* Aktivität verändern insbesondere vermindern oder eliminieren. Des weiteren beschreibt die Erfindung die Verwendung von transgenen C. *elegans,* die ein erfindungsgemäßes Nukleinsäuremolekül aufweisen, in einem Verfahren zur Identifizierung und/oder Charakterisierung von Substanzen, die die *hop-1* oder *sel-12* Aktivität steigern.

Zudem beschreibt die Erfindung die Verwendung ebensolcher transgener *C. elegans* in einem in einem Verfahren zur Identifizierung und/oder Charakterisierung von Substanzen, die als Wirkstoffe zur Behandlung und/oder Prävention der Alzheimerschen Krankheit verwendet werden können. Im Hinblick auf die Analogie zu humanen Zellen und im Hintergrund zur gezeigten funktionalen Ähnlichkeit des Notch Signalwegs und der Presenilinfunktion in *C. elegans* und Mensch wird davon ausgegangen, dass die Fehlfunktion der FAD Presenilinmutanten äquivalent durch Mutation eines homologen oder funktional äquivalenten Gens zu *ses-1* im Mensch kompensiert werden kann. Im Rahmen der Erfindung wird daher erwartet, dass ein Defekt in einem humanen ses-1 Homologen oder funktional äquivalenten Gen zu einer Steigerung der Expression des nicht von der FAD Mutation betroffenen Presenilin-Gens führt.

Die Erfindung beschreibt daher auch die Verwendung von Substanzen, die die Expression eines humanen Presenilin steigern, zur Behandlung der Alzheimer'schen Krankheit, sowie diese Substanzen selbst. Insbesondere sind das solche Substanzen, die das humane Presenilingen 1 oder 2 aktivieren und die Expression des humanen Presenilinproteins 1 oder 2 steigern. Zu den Substanzen gehören auch jene, die in *C. elegans* zu einer Veränderung der *ses-1* Aktivität führen, und/oder die *sel-12* oder *hop-1* Presenilin Aktivität steingern. Beschrieben sind auch pharmazeutische Zusammensetzungen, die diese Substanzen enthalten.

Analog kann diese Erfindung auch bei der Behandlung der sporadischen, altersabhängigen und nicht an Mutationen gekoppelten Fälle der Alzheimer'schen Erkankung von Nutzen sein. Dabei wird erwartet, dass die Minderung der Expression durch eine Steigerung der Presenilinaktivität die Bildung von Aβ4, insbesondere von Aβ42, vermindert und dadurch der Ausbruch der Erkrankung zeitlich verzögert wird.

### Kurze Beschreibung der Figuren und des Sequenzprotokolls

- Fig. 1:: *Ses-1* Mutationen
- Fig. 2:: Northern Blot, hybridisiert mit einer *hop-1* spezifischen Probe
- Fig. 3:: 5' Ende der gefundenen *ses-1* mRNAs
- Fig. 4:: Domänenstrukturvorhersage für SES-1 Protein

- SEQ ID NR:1:: SES-1 Aminosäuresequenz, lange Form
- SEQ ID NR:2:: SES-1 Aminosäuresequenz, kurze Form
- SEQ ID NR:3:: cDNA, die für die lange Form des SES-1 Proteins codiert, von ATG bis stop (yk64e9)

- SEQ ID NR:4:: cDNA, die für die kurze Form des SES-1 Proteins codiert, von ATG bis stop (yk247e5)
- SEQ ID NR:5:: cDNA, die für die lange Form des SES-1 Proteins codiert, von ATG bis stop (yk64e9) + nichtkodierende Region 3'Ende
- SEQ ID NR:6:: cDNA, die für die lange Form des SES-1 Proteins codiert, von ATG bis stop (yk64e9) + nichtkodierende Region 3'Ende
- SEQ ID NR:7:: Sequenz von pBy1015, dieser Abschnitt komplementiert den Defekt der *ses-1* Nullmutante

Die Erfindung wird im folgenden anhand von Beispielen näher erläutert.

### Experimenteller Teil

### Isolierung von ses-1 Mutanten

EMS und UV/TMP Mutagenese wurde nach publizierten Methoden durchgeführt (elegans.swmed.edu). Um *sel-12* Suppressoren zu identifizieren, wurden *sel-12* Mutanten des L4 Stadiums dem Mutagen (z.B. Ethylmethansulfonat) exponiert und nach einer Rekonvaleszenzzeit in Gruppen zu 4 oder 5 auf 9 cm Petrischalen ausgebracht. In allen Tests wurde im F2 Stadium nach der Mutagenese die Eiablage der Tiere analysiert. *Sel-12* Nullmutanten legten normalerweise keine Eier. Die F3 Generation der isolierten Tiere wurde erneut getestet und solche Tiere vereinzelt, die in beiden Generationen mindestens 5 Eier pro adultem Tier legten. Auf diese Weise wurden homozygote Suppressorstämme generiert. Alle Mutationen wurden zur Entfernung von Hintergrundmutationen mehrmals mit Wildtyptieren rückgekreuzt. Starke Suppressormutationen führten zu einem Eiablageverhalten der Tiere, das trotz der homozygoten *sel-12* Nullmutante vergleichbar war mit jenem der Wildtyptiere. Eine solche Suppressormutation wurde *ses-1*-Mutation genannt. Typischerweise legten *ses-1 sel-12* Doppelmutanten ca. 250-350 Eier pro Tier.

### Der ses-1 Phänotyp

Mutationen in *ses-1* supprimieren den Eiablagedefekt von *sel-12* Mutanten vollständig. Unter dem Stereomikroskop können 3 Aspekte des *sel-12* Eiablagedefekts erkannt werden. Etwa 75 % aller *sel*-*12*(ar171) Tiere zeigen eine deutliche Ausbuchtung der Vulva, die als protruding vulva Phänotyp bekannt ist (Pvl). Die genaue Ursache dieses Defekts ist nicht beschrieben. Praktisch alle *sel-12*(ar171) Tiere enthalten mehr Eier im Uterus als vergleichbare Wildtyptiere, deren konstante Eizahl ca. 15 beträgt, während dies bei *sel-12* bis zu 60 Eier sind. Dies führt zu einem Aufblähen der Tiere, dem beschriebenen Egl Phänotyp. Die Eier akkumulieren sich im Uterus, Embryos schlüpfen intrauterin und entwickeln sich weiter im Muttertier, das in praktisch 100 % aller Fälle vorzeitig dabei stirbt. Der Phänotyp heißt "bag of worms" Phänotyp. Wildtyptiere werden in homozygoten *sel-12* Kulturen außerordentlich selten beobachtet. *Ses-1* Mutationen supprimieren alle drei Aspekte dieses *sel-12*(ar171) Phänotyps nahezu vollständig. *Sel-12*(ar171); *ses-1* Tiere sind geringfügig kleiner als Wildtyptiere und enthalten weniger Eier im Uterus als gewöhnlich (ein schwacher Eglkonstitutiver Phänotyp). Dies lässt den Schluss zu, dass Eiablage in den Doppelmutanten geringfügig überstimuliert ist. Ses-1 allelische Tiere supprimieren auch die Eiablagedefekte aller anderen *sel-12* Mutationen, die beschrieben wurden. Dies beweist, dass *ses-1* Mutationen nicht allelspezifisch wirken.

### Klonierung des ses-1 Genlocus

*Ses-1* wurde auf dem linken Arm des Chromosoms X kartiert. Dazu wurden *sel-12(*ar171) *ses-1* Stämme über einer genetischen Markermutation platziert, Eiablagedefekte (Egl) rekombinante. Tiere wurden isoliert und es wurde getestet, wie viele dieser Tiere die Markermutation übernommen hatten. Um *ses-1* näher zu kartieren, wurde die Gegenwart/Abwesenheit von *ses-1* Mutationen in einem Stamm durch die Suppression des *sel-12*(ar171) Eiablagedefekts gemessen. Dazu wurden *sel-12*(ar171) *ses-1* Männchen mit AB Doppelmutantenstämmen gekreuzt, wobei A und B zwei Mutationen sind, die rechts von *sel-12* auf der genetischen Karte lokalisiert sind und wobei B der rechte der beiden Marker ist. Es wurden dann nur B Rekombinanten gesucht, die nicht die A Mutation trugen. Durch diese Rekombinationsmethode sollten alle Rekombinanten *sel-12*(ar171) enthalten, außer denen, die eine Doppelrekombination trugen. Durch eine Reihe von Kreuzungsexperimenten wurde *ses-1* zwischen dpy-23(e840) und lin-2(e678), ca. 1/5 der Distanz von dpy-23(e840) zu lon-2, kartiert. Alle Experimente wiesen darauf hin, dass *ses-1* Mutationen Funktionsverlust bewirken. Um dies nachzuweisen, wurden *ses-1* Mutationen über zwei freie Duplikationen platziert. Obwohl die *ses-1* Allele by108 und by109 Mutationen einander nicht komplementieren, bestand die Möglichkeit, dass beide Mutationen in zwei unterschiedlichen Genen auf dem linken Arm des X Chromosoms repräsentieren. Daher wurden anfänglich by108 und by109 unabhängig voneinander kartiert. Um die Kartierungen zu verifizieren wurden zwei Duplikationen, mnDp31 und mnDp32, in den Stamm *sel-12*(ar171) *ses-1*(by108) lon-2(e678) transferiert und Eiablage getestet.
Der Stamm *sel-12*(ar171) *ses-1*(by108) lon-2(e678); nDp31, der zwei mutierte Kopien des *ses-1* Gens auf dem X Chromosom trägt sowie eine Wildtypkopie auf einer extrachromosomalen Array-DNA besitzt, besitzt gänzlich *ses-1* Wildtyp Phänotyp, was beweist, daß die getesteten Mutanten Funktionsverlustmutanten sind. Durch lineare Interpolation der Daten wurde *ses-1* ca. 50 kb links von dpy-23(e840) lokalisiert, und nachfolgend wurde die transgene Suppression durch Cosmide dieser Chromsomenregion getestet.

### Transgene Rettung des ses-1 Defekts

Es wurden Transgene in den Stamm *sel-12*(ar171) *ses-1*(by108) injiziert und die Antisuppressor-Aktivität der transfizierten Klone getestet. Alle Klone wurden bei einer Konzentration von 20 ng/µl mit 100 ng/µl pRF4 und 20 ng/µl pBY218 Transformationsmarkern injiziert. Der *ses-1* Phänotyp wurde durch Injektion des Cosmids F46H6 vollständig kompensiert (gerettet). Danach wurden F46H6 Subklone , gereinigte Restriktiensfragmente, sowie PCR Produkte dieses Cosmids getestet, um die Identität des *ses-1* Gens aufzuklären.

### Isolierung von F46H6 Subfragmenten

F46H6 wurde mit verschiedenen Restriktionsenzymen geschnitten. Subfragmente wurden mit Qiaquick Gelextraktionskits gereinigt. Ein 21.5 kb NcoI und ein 9.2 kb BamHI Fragment (im Plasmid pBY1015) wurden direkt injiziert und retten den Phänotyp (beide). Das minimale rettende Fragment wurde auf 4 kb Sequenz verkleinert. Dieser Abschnitt enthält nur ein Gen, das für ein Protein mit zahlreichen Zinkfingern kodiert.

### Struktur von ses-1

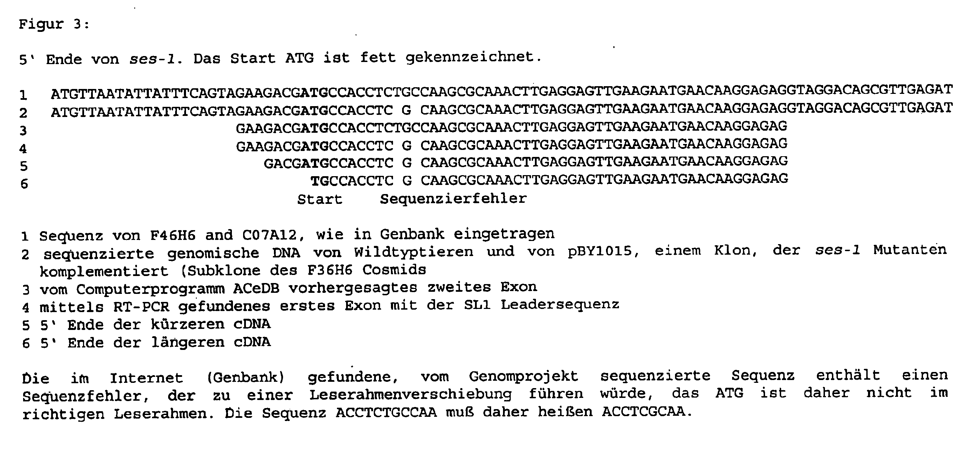

Zwei cDNAs von *ses-1* wurden identifiziert und enthielten 1.9 kb und 2.1 kb Sequenz. Beide cDNAs enthalten identische DNA-Sequenzen und Exon-Intron-Grenzen mit einer Ausnahme: Der größere Klon trägt ein zusätzliches Intron, der kleine hat ein um 6 Basen längeres 5' Ende (siehe dazu Figur 3).
Die sequenzierten cDNAs differieren von dem in den Datenbanken vorhergesagten Gen an mehreren Stellen. Zum einen ist das gefundene 5' Ende deutlich kürzer als durch Computer analyse vorhergesagt. Zum zweiten wurden Sequenzunterschiede in zwischen beiden cDNAs und den im Internet veröffentlichen Genomsequenzen der Region auf den Cosmiden F46H6 und C07A12(Nachbar) gefunden (Figur 3).
*Ses-1* wird außerdem an eine SL1 Leadersequenz transgespleißt. Das SL1 spezifische Produkt wurde sequenziert. Es wurde gefunden, dass die Leadersequenz unmittelbar 5' an die beiden vorhergesagten 5' Enden der cDNAs anschließt.
Nur für die längere cDNA wurde in Northern Blot Experimenten eine Bande gefunden, die in allen Entwicklungsstadien von C. *elegans* detektierbar ist. Entweder könnte die kürzere cDNA ein Artefakt sein oder sie tritt nur in kleinen, undetektierbaren Mengen auf, könnte aber durchaus biologische Aktivität aufweisen.

### Intrazelluläre Lokalisation

Ein *ses-1* Fusionsgen wurde in einem Bacculovirus SF9 Zellsystem exprimiert und durch Immunofärbung die Lokalisation nachgewiesen. *Ses-1* ist kernlokalisiert, dies stimmt mit der prognostizierten Rolle eines Transkriptionsfaktors überein.

### Expression

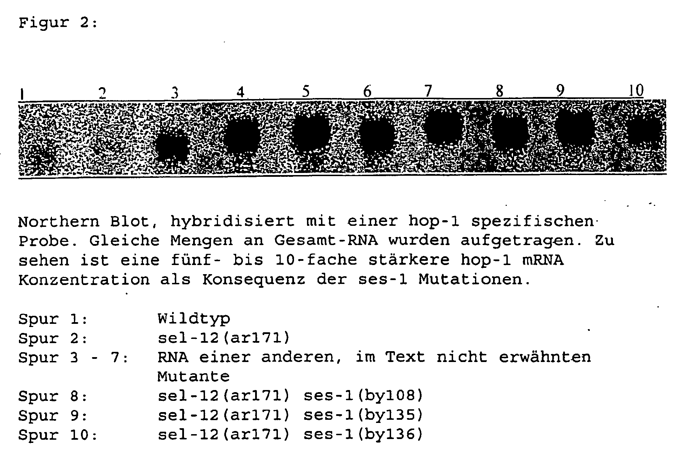

Die Northern Blot Analysen zeigten, dass *ses-1* Transkripte in allen Entwicklungsstadien exprimiert werden (Figur 2).

### Der ses-1 Null Phänotyp

By131 repräsentiert eine Mutante, die keinerlei *ses-1* Funktion mehr haben kann (Deletion des gesamten Gens). Allerdings werden durch diese Deletion auch Nachbargene eliminiert. By135 führt zu einer Leserahmenverschiebung, wie beschrieben, und sollte daher die stärkste *ses-1* Mutante darstellen. *Ses-1*(by135) und by131 Tiere wurden untersucht. Beide Stämme sind phänotypisch Wildtyp, sowohl morphologisch als auch aufgrund ihres Eiablageverhaltens. Die Nachkommenzahl beider Tiere ist etwas niedriger als im Wildtyp. Beide Allele supprimieren den *sel-12* Phänotyp aller Allele vollständig und führen auch zu Wildtyp Nachkommenzahlen der *sel-12* Allele. Somit kann die Schlussfolgerung gezogen werden, dass *ses-1* ein spezifischer Suppressor von *sel-12* ist.
Alle Defekte von *sel-12* Mutanten oder von *sel-12, hop-1* Doppelmutanten können auf Effekte dieser Preseniline im Notch Signalwege von lin-12 und glp-1 zurückgeführt werden.

Da *ses-1* ein nukleär exprimiertes Gen ist, das als Transkriptionsfaktor wirken könnte, wurde untersucht, ob die *ses-1* Mutationen die Transkription anderer Gene des lin-12/glp-1 Notch Signalwegs verändern können. Stadienspezifische RNAs des Wildtyp und verschiedener *ses-1* Mutantenstämme wurden isoliert und im Northern Blot analysiert. Die Proben wurden durch Hybridisierung mit *hop-1* cDNAs getestet. *Hop-1* wird in Wildtyptieren in den Eiern stark exprimiert, aber ist im L1 Larvenstadium praktisch nicht nachweisbar. Die Expression steigt vom L2 zum adulten Stadium langsam an und erreicht ihren Höhepunkt in adulten Tieren. In *sel*-*12*(ar171) Tieren war die Expression von *hop-1* weitgehend identisch. In se*l*-*12*(ar171) *ses-1*(by135) Tieren und *ses-1*(by135) Tieren unterschied sich die Expression in einem Stadium signifikant: *hop-1* war deutlich im L1 Stadium nachweisbar. Auch in den *ses-1* Allelen by108 und by136 war die Expression von *hop-1* im L1 Stadium signifikant stärker.
Dies bedeutet, dass *ses-1* Mutanten die Suppression von *sel-12* Defekten dadurch erreichen, dass sie die Expression von *hop-1* deutlich dereprimieren und HOP-1 Protein vermutlich die Funktion des defekten SEL-12 übernehmen kann.

## Patentansprüche

1. isoliertes Nukleinsäuremolekül, das für ein Protein codiert, welches die Expression von hop-1 reprimiert und/oder dessen Ausschaltung eine gesteigerte Expression von hop-1 bewirkt, wobei das Nukleinsäuremolekül die in SEQ ID Nr. 3, SEQ ID Nr. 4, SEQ ID Nr. 5 oder SEQ ID Nr. 6 angegebene Nukleotidsequenz umfasst.

2. Isoliertes Nukleinsäuremolekül nach Anspruch 1, wobei das Nukleinsäuremolekül ein DNA-Molekül, insbesondere ein cDNA-Molekül oder ein genomisches DNA-Molekül, oder ein RNA-Molekül ist.

3. Isoliertes Nukleinsäuremolekül nach einem der Ansprüche 1 oder 2, wobei das Protein die identische Aminosäuresequenz wie die in SEQ ID Nr. 1 oder SEQ ID Nr. 2 gezeigte Aminosäuresequenz aufweist.

4. Isoliertes Nukleinsäuremolekül gemäß einem der Ansprüche 1 bis 3, welches eine oder mehrere der folgenden Mutationen aufweist,
a) eine Mutation, die dazu führt, dass das codierte Protein C-terminal trunkiert ist, wobei ihm zumindest die Aminosäuren ab Position 495 fehlen;
b) ein G zu A Austausch an Position +1784 der Nukleinsäure;
c) eine Mutation, die dazu führt, dass sich an Position 596 des codierten Proteins an Stelle von C ein Y befindet;
wobei die Mutation den Eiablagedefekt von *sel-12*-Mutanten behebt.

5. Isoliertes Nukleinsäuremolekül gemäß Anspruch 4, wobei die Mutation
a) eine Mutation ist, die zum Translationsstop bei Position 210, 389, 465 oder 495 des codierten Proteins führt;
b) ein C zu T Austausch an Position +1393 der Nukleinsäure ist;
c) eine Mutation, die eine Inversion der Nukleotide CAA an Position +1481 zu AAC bewirkt;
d) ein G zu A Austausch an Position +1784 der Nukleinsäure ist;
e) die Deletion eines A an Position +549 ist;
f) ein T zu A Austausch an Position +1163 der Nukleinsäure ist.

6. Vektor, welcher ein isoliertes Nukleinsäuremolekül nach einem der Ansprüche 1 bis 5 umfasst.

7. Vektor nach Anspruch 6, welcher einen Promoter für die Expression in einer Zelle des Nematoden *C*. *elegans* umfasst.

8. Vektor nach einem der Ansprüche 6 oder 7, welcher ein Plasmid, ein Cosmid, ein Bacmid, ein Virus oder ein Bakteriophage ist.

9. Isoliertes Protein, welches die Expression von hop-1 reprimiert und/oder dessen Ausschaltung eine gesteigerte Expression von hop-1 bewirkt, mit der in SEQ ID Nr. 1 oder SEQ ID Nr. 2 gezeigten Aminosäuresequenz oder codiert durch eine Nukleinsäure mit einer der Sequenzen gemäß SEQ ID NOs. 3, 4, 5, 6 oder 7.

10. Isoliertes Protein gemäß Anspruch 9, welches eine oder mehrere der folgenden Mutationen aufweist, die den Eiablagedefekt von *sel-12*-Mutanten beheben,
a) ein an Position 495, 465, 385 oder 210 C-terminal trunkiertes Protein
b) an Position 596 an Stelle von C ein Y.

11. Antikörper, der gegen ein Protein nach einem der Ansprüche 9 bis 10 hervorgerufen wurde.

12. Antikörper nach Anspruch 11, **dadurch gekennzeichnet, dass** der Antikörper ein monoklonaler oder ein polyklonaler Antikörper ist.

13. Transgener nichtmenschlicher Organismus, welcher ein Nukleinsäuremolekül nach irgendeinem der Ansprüche 1 bis 5 umfasst.

14. Transgener nichtmenschlicher Organismus nach Anspruch 13, welcher ein Nematode, insbesondere *C. elegans* ist.

15. Verwendung eines isolierten Nukleinsäuremoleküls nach einem der Ansprüche 1 bis 5 zur Herstellung eines Arzneimittels zur Behandlung neuroiogischer, insbesondere neurodegenerativer Erkrankungen.

16. Verwendung eines isolierten Nukleinsäuremoleküls nach einem der Ansprüche 1 bis 5 zur Erzeugung eines nichtmenschlichen Modellorganismus zur Untersuchung und Erforschung neurologischer, insbesondere neurodegenerativer Erkrankungen.

## Claims

1. An isolated nucleic acid molecule which encodes a protein that represses expression of hop-1 and/or whose elimination brings about increased expression of hop-1, wherein the nucleic acid molecule comprises the nucleotide sequence given in SEQ ID NO: 3, SEQ ID NO: 4, SEQ ID NO: 5, or SEQ ID NO: 6.

2. The isolated nucleic acid molecule as claimed in claim 1, wherein the nucleic acid molecule is a DNA molecule, in particular a cDNA molecule or a genomic DNA molecule, or an RNA molecule.

3. The isolated nucleic acid molecule as claimed in either of claims 1 and 2, wherein the protein has an amino acid sequence which is identical to the amino acid sequence shown in SEQ ID NO: 1 or SEQ ID NO: 2.

4. The isolated nucleic acid molecule as claimed in one of claims 1 to 3, comprising one or more of the following mutations:
a) a mutation causing the encoded protein to be C-terminally truncated, it is lacking at least the amino acid from position 495 onward;
b) a G to A exchange at position +1784 of the nucleic acid;
c) a mutation causing there to be a Y in place of C at position 596 of the encoded protein;
the mutation remedying the egg-laying defect of *sel-12* mutants.

5. The isolated nucleic acid molecule as claimed in claim 4, wherein the mutation
a) is a mutation leading to translation stop at position 210, 389, 465 or 495 of the encoded protein;
b) is a C to exchange at position +1393 of the nucleic acid;
c) a mutation which causes an inversion of the nucleotides CAA at position +1481 to AAC;
d) is a G to A exchange at position +1784 of the nucleic acid;
e) is a deletion of an A at position +549;
f) is a T to A exchange at position +1163 of the nucleic acid.

6. A vector which comprises an isolated nucleic acid molecule as claimed in one of claims 1 to 5.

7. The vector as claimed in claim 6, which comprises a promoter for expression in a cell of the nematode *C. elegans.*

8. The vector as claimed in either of claims 6 and 7, which is a plasmid, a cosmid, a bacmid, a virus or a bacteriophage.

9. An isolated protein that represses expression of hop-1 and/or whose elimination brings about increased expression of hop-1, having the amino acid sequence shown in SEQ ID NO: 1 or SEQ ID NO: 2, or encoded by a nucleic acid having one of the sequences of SEQ ID NOs: 3, 4, 5, 6 or 7.

10. The isolated protein as claimed in claim 9 which comprises one or more of the following mutations which remedy the egg-laying defect of *sel-12* mutants:
a) a protein C-terminally truncated at position 495, 465, 385 or 210
b) a Y instead of C at position 596.

11. An antibody which has been produced against a protein as claimed in one of claims 9 to 10.

12. The antibody as claimed in claim 11, wherein the antibody is a monoclonal antibody or a polyclonal antibody.

13. A transgenic, nonhuman organism which comprises a nucleic acid molecule as claimed in any one of claims 1 to 5.

14. A transgenic, nonhuman organism as claimed in claim 13 which is a nematode, in particular C. *elegans.*

15. The use of an isolated nucleic acid molecule as claimed in one of claims 1 to 5 for producing a pharmaceutical for treating neurological, in particular neurodegenerative, diseases.

16. The use of an isolated nucleic acid molecule as claimed in one of claims 1 to 5 for generating a nonhuman model organism for the investigation and elucidation of neurological, in particular neurodegenerative, diseases.

## Revendications

1. Molécule d'acide nucléique isolée qui code une protéine qui réprime l'expression du hop-1 et/ou dont l'extinction provoque une expression accrue du hop-1, la molécule d'acide nucléique comprenant la séquence de nucléotides données dans la SEQ ID N° 3, SEQ ID N° 4, SEQ ID N° 5 ou SEQ ID N° 6.

2. Molécule d'acide nucléique isolée selon la revendication 1, dans laquelle la molécule d'acide nucléique est une molécule d'ADN, en particulier une molécule d'ADNc ou une molécule d'ADN génomique, ou une molécule d'ARN.

3. Molécule d'acide nucléique isolée selon l'une des revendications 1 ou 2, dans laquelle la protéine présente la séquence d'acides aminés identique à la séquence d'acides aminés représentée dans la SEQ ID N° 1 ou la SEQ ID N° 2.

4. Molécule d'acide nucléique isolée selon l'une des revendications 1 à 3, qui présente une ou plusieurs des mutations suivantes:
a) une mutation qui fait que la protéine codée est tronquée à son extrémité C-terminale, les acides aminés au moins à partir de la position 495 manquant ;
b) un échange G à A en position +1784 de l'acide nucléique ;
c) une mutation qui fait qu'on trouve un Y à la position 596 de la protéine codée en lieu et place de C;
la mutation éliminant l'absence de ponte d'oeufs des mutants *sel-12.*

5. Molécule d'acide nucléique isolée selon la revendication 4, dans laquelle la mutation :
a) est une mutation qui amène un arrêt de translation à la position 210, 389, 465 ou 495 de la protéine codée ;
b) est un échange C à T en position +1393 de l'acide nucléique ;
c) est une mutation qui provoque l'inversion des nucléotides CAA en position +1481 en AAC ;
d) est un échange G à A en position +1784 de l'acide nucléique ;
e) est l'élimination d'un A en position +549 ;
f) est un échange T à A en position +1163 de l'acide nucléique.

6. Vecteur comprenant une molécule d'acide nucléique isolée selon l'une des revendications 1 à 5.

7. Vecteur selon la revendication 6, qui comprend un promoteur de l'expression dans une cellule du nématode C. *elegans.*

8. Vecteur selon l'une des revendications 6 ou 7, qui est un plasmide, un cosmide, un bacmide, un virus ou un bactériophage.

9. Protéine isolée qui réprime l'expression de hop-1 et/ou dont l'extinction provoque une expression accrue du hop-1 avec la séquence d'acides aminés représentée dans la SEQ ID N° 1 ou la SEQ ID N° 2 ou codée par un acide nucléique ayant l'une des séquences selon la SEQ ID N° 3, 4, 5, 6 ou 7.

10. Protéine isolée selon la revendication 9, laquelle présente une ou plusieurs des mutations suivantes, qui éliminent l'absence de ponte d'oeufs des mutants *sel-12,*
a) une protéine tronquée à l'extrémité C-terminale en position 495, 465, 385 ou 210,
b) un Y en position 596 en lieu et place de C.

11. Anticorps suscité contre une protéine selon l'une des revendications 9 à 10.

12. Anticorps selon la revendication 11, **caractérisé en ce que** l'anticorps est un anticorps monoclonal ou polyclonal.

13. Organisme transgénique non humain comprenant une molécule d'acide nucléique selon l'une quelconque des revendications 1 à 5.

14. Organisme transgénique non humain selon la revendication 13, qui est un nématode, en particulier C. *elegans.*

15. Utilisation d'une molécule d'acide nucléique isolée selon l'une des revendications 1 à 5 pour la préparation d'un médicament de traitement de maladies neurologiques, en particulier neurodégénératives.

16. Utilisation d'une molécule d'acide nucléique isolée selon l'une des revendications 1 à 5 pour la production d'un organisme modèle non humain destiné à l'analyse et à la recherche sur les maladies neurologiques, en particulier neurodégénératives.
